(19) Europäisches Patentamt
European Patent Office
Office européen des brevets

(11) Publication number: 0 211 584 A1

# (12) EUROPEAN PATENT APPLICATION

(21) Application number: 86305734.5

(22) Date of filing: 25.07.86

(51) Int. Cl.⁴: **C07D 471/04** , A61K 31/435 , //(C07D471/04,221:00,221:00)

(30) Priority: **29.07.85 US 760220**
**17.12.85 US 809962**
**26.02.86 GB 8604782**

(43) Date of publication of application: **25.02.87 Bulletin 87/09**

(84) Designated Contracting States: **AT BE CH DE FR IT LI LU NL SE**

(71) Applicant: **AMERICAN HOME PRODUCTS CORPORATION**
**685, Third Avenue**
**New York, New York 10017(US)**

(72) Inventor: **Sulkowski, Theodore Sylvester**
**316 Rockland Road**
**Wayne Pennsylvania(US)**
Inventor: **Silver, Paul Joseph**
**4 Primrose Lane**
**West Chester Pennsylvania(US)**
Inventor: **Mascitti, Albert Angelo**
**1602 Daws Road**
**Norristown Pennsylvania(US)**

(74) Representative: **Wileman, David Francis et al**
**c/o John Wyeth and Brother Limited**
**Huntercombe Lane South**
**Taplow Maidenhead Berkshire SL6 OPH(GB)**

## (54) Antihypertensive agents.

(57) The invention describes antihypertensive agents of formula

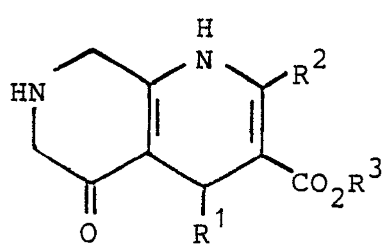

(I)

in which $R^1$ is a phenyl group substituted by defined substituents, $R^2$ is $C_1$-$C_6$ alkyl, $R^3$ is a group of formula

EP 0 211 584 A1

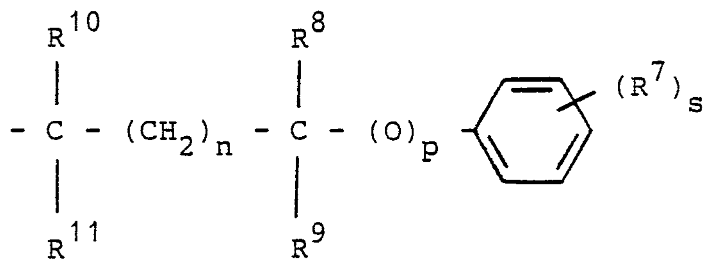

where $R^7$ is independently hydrogen or defined substituents,

$R^8$ is hydrogen, $C_1$-$C_6$ alkyl or $C_1$-$C_6$ alkoxy,

$R^9$, $R^{10}$ and $R^{11}$ are independently hydrogen or $C_1$-$C_6$ alkyl,

n is 0,1,2,3 or 4; s is 1,2,3,4 or 5;

p is 0 or 1 providing that when $R^8$ is $C_1$-$C_6$ alkoxy p is 0,

or $R^3$ is

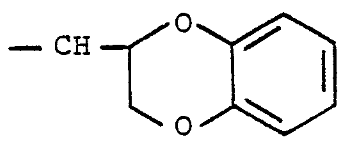

or

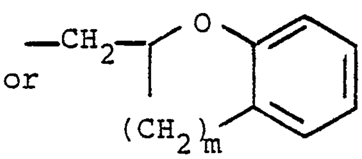

where m is 1,2,3 or 4 or a pharmaceutically acceptable salt thereof.

Also described are processes for preparing the compounds, intermediates used in such processes and pharmaceutical compositions containing the active compounds.

## ANTIHYPERTENSIVE AGENTS

This invention relates to antihypertensive agents, more particularly to naphthyridine derivatives, processes for preparing them pharmaceutical compositions containing them and intermediates of analogous structure for preparing them.

Pharmacological agents possessing the ability to block cellular transmembrane influx of calcium are capable of suppressing that portion of myocardial or vascular smooth muscle contractility which is dependent upon extracellular calcium. Church et al., Can.J.Physiol.Pharmacol., 58,254 (1980); Fleckenstein, Calcium and the Heart, P.Harris and L.Opie, eds., Acadamic Press (1971); Nayler et al., Bas.Res.Cardiol., 76, 1 (1981); Calcium Blockers, S.Flaim and R.Zelis, eds., Urban and Schwartzenberg, (1982).

These pharmacological agents, termed calcium entry blockers, have been proven to be useful in the treatment of hypertension, cardiac arrhythmias, angina pectoris, and coronary artery vasospasm (a possible cause of sudden cardiac death syndrome). Circ. Res., 52, Suppl. I (1983); Hypertension 5, Suppl.II (1983). However, a major limitation and deleterious side-effect for use of some of these agents in certain vascular pathologies is the negative inotropism associated with blockade of cardiac sarcolemmal $Ca^{+2}$ channels.

In theory, calcium entry blockers are thought to act by blocking calcium influx through discrete calcium channels (slow channels) in cell membranes. Various tissues exhibit relative differences in sensitivity toward the calcium blocking effect achieved by certain calcium antagonists, theoretically as a result of tissue specific differences in the calcium channels. Acta Pharmacol. Toxicol., 43, 5 - (1978); loc.cit 291 (1978); Microvascular Res., 5, 73 (1973); Am. Rev. Pharmacol. Toxicol., 17, 149 - (1977).

A mechanistic difference in $Ca^{+2}$ regulation of contractile activity in vascular smooth muscle and cardiac muscle is believed to exist. In cardiac muscle, $Ca^{+2}$ regulation is primarily thin filament-linked and involves the troponin-tropomyosin system. Stull et al., Handbook of Physiology, The Cardiovascular System, vol. 1, R.Berne, N.Sperelakis and S.Geiger, eds., American Physiological Society - (1979); Solaro, Calcium Blockers, ibid., supra . In vascular smooth muscle, regulation is primarily dependent upon $Ca^{+2}$-calmodulin mediated myosin light chain phosphorylation. Hartshorne et al., Handbook of Physiology, The Cardiovascular System, vol. 2., Bohr, Somlyo and Sparks, eds., American Physiological Society (1982); Silver et al., Calcium Blockers, ibid., supra.

Calcium antagonists which antagonise the effects of $Ca^{+2}$ by inhibiting $Ca^{+2}$-calmodulin mediated myosin light chain phosphorylation would be more specific for vascular smooth muscle and would be less liable to produce negative inotropic cardiac contraction.

U.S. Patents 4,321,384, granted March 31, 1982, and 4,365,063 granted December 21, 1982, disclose hexahydro-2-alkyl-4-aryl-5-oxo-1,7-naphthyridine-3-carboxylic acid esters in which the alcohol derived portion of the ester is an alkyl, alkoxyalkyl, trifluoromethylalkyl or aminoalkyl moiety.

U.S. Patent No. 4,551,534 claims hexahydro-1,7-naphthyridine derivatives in which the nitrogen in 7-position has been variously modified. The compounds disclosed in US 4,551,534 are less potent antihypertensive agents when administered orally than the modified esters of the following disclosure.

In accordance with this invention there is provided a group of 1,4,5,6,7,8-hexahydro-2-alkyl-4-aryl-5-oxo-1,7-naphthyridine-3-carboxylic acid ester derivatives and pharmaceutically acceptable acid addition salts thereof, which are useful antihypertensive agents. The compounds of this invention differ structurally from the compounds of the U.S. patents cited, supra , in the presence of an aromatic group in the alcohol moiety of the ester grouping. In addition, the compounds disclosed herein differ in their pharmacological profile in that they are more vascular specific as opposed to cardiac specific antihypertensive agents and they present less toxic liability. The compounds of this invention also antagonise arterial actin-myosin interactions and $Ca^{+2}$-calmodulin dependent myosin light chain phosphorylation. Thus, the compounds of this invention are aryloxyalkyl esters, alkoxy-(aryl)alkyl esters, arylalkyl esters, benzodioxan-2-alkyl esters or benzoalkyleneoxid-2-ylalkyl esters. The aromatic ring of the ester may be substituted by from one to five halo groups (e.g., -Cl, -Br, -F), and/or from 1 to 3 groups $-CF_3$, alkyl of 1 to 6 carbon atoms or alkoxy of 1 to 6 carbon atoms. The alkyl moiety of the ester may be straight or branched chain and contains from two to six carbon atoms in its linear portion.

More specifically, the antihypertensive agents of this invention are compounds of the formula:

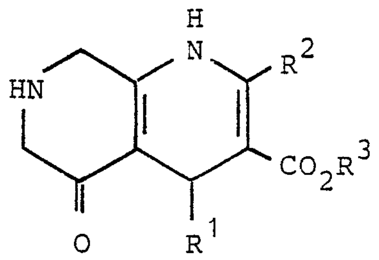

(I)

in which

$R^1$ is tetra-or penta-chloro, bromo or fluoro-phenyl or

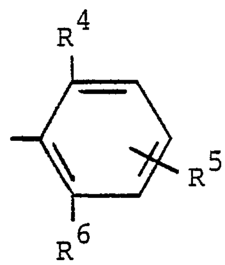

where $R^4$ and $R^6$ are, independently, hydrogen, alkyl of 1 to 6 carbon atoms, halo, trifluoromethyl, alkoxy of 1 to 6 carbon atoms, alkylthio of 1 to 6 carbon atoms, cyano or nitro; and $R^5$ is hydrogen, alkyl of 1 to 6 carbon atoms, halo, trifluoromethyl, cyano or nitro;

$R^2$ is alkyl of 1 to 6 carbon atoms; and

$R^3$ is

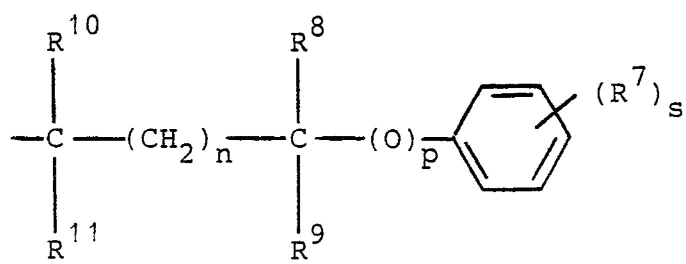

where $R^7$ is, independently, hydrogen, -Cl, -Br, -F, and no more than three of the groups $-CF_3$, alkyl of 1 to 6 carbon atoms or alkoxy of 1 to 6 carbon atoms;

$R^8$ is hydrogen, alkyl of 1 to 6 carbon atoms or alkoxy of 1 to 6 carbon atoms;

$R^9$, $R^{10}$ and $R^{11}$ are, independently, hydrogen or alkyl of 1 to 6 carbon atoms;

n is one of the integers 0,1,2,3 or 4;

s is one of the integers 1,2,3,4 or 5;

p is one of the integers 0 or 1, with the proviso that when $R^8$ is alkoxy, p is 0;

or $R^3$ is

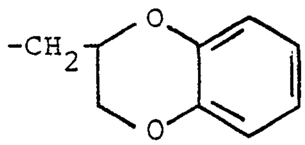

or

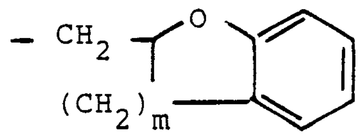

;

where m is one of the integers 1,2,3 or 4;

or a pharmaceutically acceptable salt thereof;

With reference to the above-described compound genus, the preferred variables from the standpoint of production economics and activity profile are those in which the group $R^1$ contains halogen substituents and/or $R^3$ is an aryloxyalkyl group devoid of chiral centres. Thus, the preferred subgenus is a group of compounds of the formula I in which

$R^1$ is tetra-or penta-chloro, bromo or fluoro-phenyl or

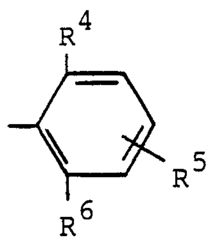

where $R^4$ and $R^6$ are halo and $R^5$ is defined above;

$R^2$ is alkyl of 1 to 6 carbon atoms; and

$R^3$ is

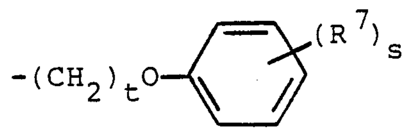

where t is one of the integers 2,3,4,5 or 6 and $R^7$ and s are defined above; or a pharmaceutically acceptable salt thereof.

A second subgeneric group of compounds of this invention have formula I in which $R^1$ and $R^2$ are as previously defined in the generic statement of the invention, and $R^3$ is

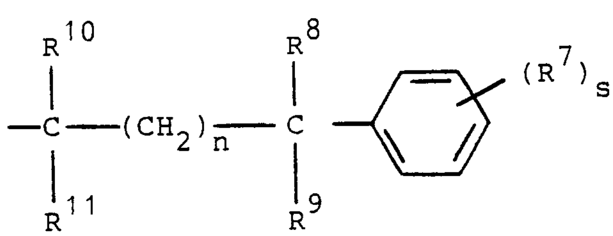

where n,s,$R^7$,$R^9$,$R^{10}$ and $R^{11}$ are as previously defined and $R^8$ is alkoxy of 1 to 6 carbon atoms; or a pharmaceutically acceptable salt thereof.

The aromatic alkylene oxide subgenus of compounds of this invention have formula I in which $R^1$ and $R^2$ are as defined in the generic statement of the invention and $R^3$ is

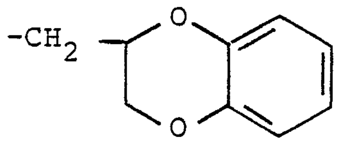

or

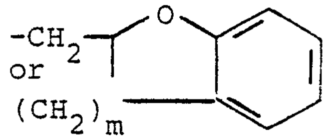

where m is one of the integers 1,2,3 or 4 or a pharmaceutically acceptable salt thereof.

Examples of $R^1$ are halosubstituted phenyl groups such as 3-chlorophenyl, 2,3-dichlorophenyl,2,3,6-trifluorophenyl, 2-chloro-6-fluorophenyl and 2,3-dichloro-6-fluorophenyl; perhalo substituted phenyl groups such as pentafluorophenyl; phenyl groups substituted by halogen and $C_1$-$C_6$-alkyl e.g. 3-chloro-2-methylphenyl or 2-chloro-6-fluoro-3-methylphenyl, phenyl groups substituted by $CF_3$ e.g. 2-trifluoromethylphenyl and phenyl groups substituted by $C_1$-$C_6$ alkyl e.g. 2-methylphenyl.

$R^2$ may be an alkyl group such as methyl, ethyl or propyl. Preferably $R^2$ is methyl.

Examples of $R^3$ are (i) groups of formula

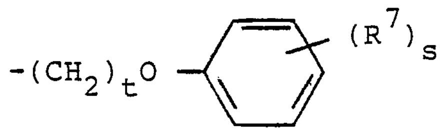

where t is an integer from 2 to 6 and $R^7$ and s are as defined above, such as 2-phenoxyethyl, 4-phenoxybutyl and 2-(2,3-dichlorophenoxy)ethyl; and

(ii) groups of formula

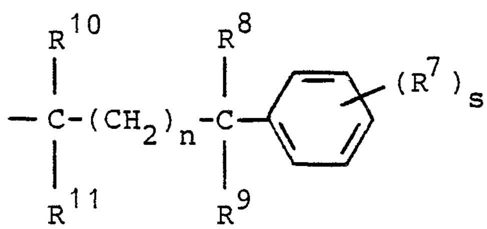

where n,s,$R^7$,$R^9$,$R^{10}$ and $R^{11}$ are as previously defined and $R^8$ is alkoxy of 1 to 6 C atoms, such as 2-methoxy-2-phenylethyl (i.e. n is 0). Accordingly preferred examples of t are 2 and 4; preferred examples of $(R^7)_s$ are hydrogen and dichloro; $R^9$, $R^{10}$ and $R^{11}$ are preferably hydrogen; $R^8$ is preferably methoxy. Other examples of $R^3$ are 1-methyl-2-phenoxyethyl, 2-methyl-2-phenoxypropyl and 2,3-dihydro-1,4-benzodioxin-2-ylmethyl.

This invention also provides processes for preparing the compounds of formula I as defined above. A first process comprises deprotecting a compound of formula II

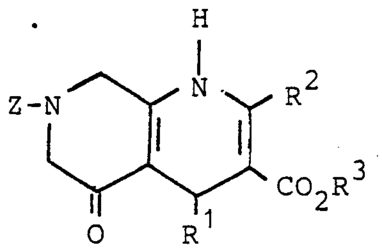

(II)

wherein $R^1$, $R^2$ and $R^3$ are as defined above and Z is a methyl group substituted by up to 3 aryl groups (preferably aryl of 6 to 10 carbon atoms) and if desired isolating the product as the free base or an acid addition salt.

Examples of the protecting group Z are benzyl, diphenylmethyl and trityl, most preferably Z is benzyl. The protecting group is conveniently removed by hydrogenolysis under standard conditions preferably in the presence of a mineral acid and using an appropriate catalyst such as a platinum metal catalyst e.g. palladium or platinum on carbon or a nickel catalyst e.g. Raney nickel. Preferably the catalyst is palladium on carbon.

For a description of methods for carrying out hydrogenolysis see standard textbooks such as "Practical Catalytic Hydrogenation", M. Freifelder, Wiley Interscience, 1971 especially pages 413-428; "Catalytic Hydrogenation over Platinum Metals", P.Rylander, Academic Press, New York and London 1967 and Organic Reactions, Volume VII p 263-326 (1953).

A second process for preparing compounds of formula I comprises transesterifying a compound of formula

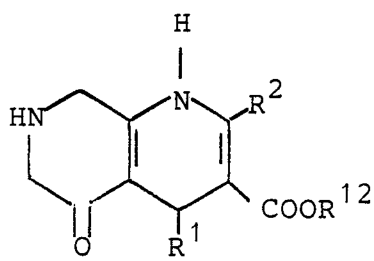

III

where $COOR^{12}$ is an ester group other than that desired in the end product. The transesterification may be carried out by treating the starting ester of formula III with an alcohol of formula $R^3OH$ preferably in excess and in presence of base e.g. an alkali metal or alkali metal alkoxide e.g. Na OMe. Examples of groups for $R^{12}$ are alkyl groups especially those having 1 to 6 carbon atoms e.g. methyl or ethyl.

Transesterification reactions are illustrated in "Compendium of Organic Synthetic Methods", by I.T.Harrison and S.Harrison, Wiley Interscience, 1971 p 292.

In a preferred embodiment the compounds of this invention may be prepared by reaction of 1-benzyl-3-hydroxy-5-oxo-tetrahydropyridine, an appropriate ester of acetoacetic acid, an appropriately substituted benzaldehyde and an ammonia source such as ammonium acetate. The reaction is carried out in alcohol, preferably methanol at elevated temperature (e.g. reflux) for from two to about eighteen hours, preferably about six hours. The product is subjected to catalytic hydrogenation, preferably in the presence of a mineral acid, to remove the benzyl group present on the nitrogen atom in 7-position. Thus:

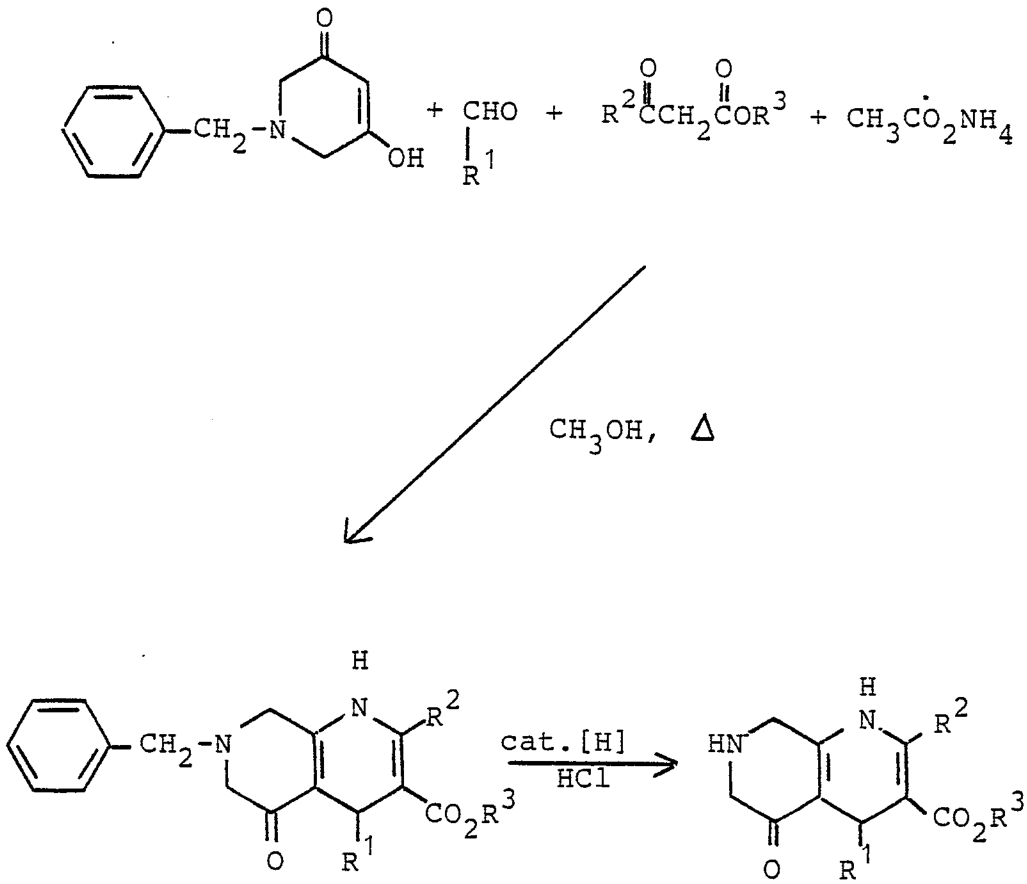


The appropriately substituted benzaldehydes are either commercially available or are prepared by standard procedures. The preparation of 2,3-dichloro-6-fluorobenzaldehyde is illustrated in Example 24, infra. The acetoacetic acid ester derivatives are conventionally prepared by reaction of diketene with the appropriately substituted alcohol. The procedure is illustrated by preparation of 2-phenoxyethyl acetoacetate:

Diketene (84g) was added dropwise with stirring to 140g of 2-phenoxy-ethanol heated at 100°C. After the addition was completed, the reaction mixture was refluxed for an additional 22 hours. The reaction mixture was fractionally distilled to obtain 90.6 g of 2-phenoxyethyl acetoacetate, BP 140-145°C/0.3 mm.

| | |
|---|---|
| Analysis for: | $C_{12}H_{14}O_4$ |
| Calculated: | C, 64.85; H, 6.35 |
| Found: | C, 64.45; H, 6.48. |

In a similar manner, diketene was reacted with 2-hydroxymethyl-1,4-benzodioxin-2-ylmethyl acetoacetate, BP 120-140°C/0.05 mm (flash distillation).

In a similar manner, diketene was reacted with 1-phenoxy-2-propanol to obtain 1-methyl-2-(phenoxy)ethyl acetoacetate, BP 115-122°C/0.05-0.1mm.

In a similar manner, diketene was reacted with (±)-2-methoxy-2-phenylethanol to obtain 2-methoxy-2-phenylethyl acetoacetate, BP 115-120°C/0.2mm.

In a similar manner diketene was reacted with 2-(2,3-dichlorophenoxy)-ethanol to obtain 2-(2,3-dichlorophenoxy)ethyl acetoacetate, BP 150-170°C/0.25mm (flash distillation).

In a similar manner, diketene was reacted with 4-phenoxybutanol to obtain 4-phenoxybutyl acetoacetate, BP 115-120°C/0.25mm (flash distillation).

In a similar manner, diketene was reacted with 2-cyclohexyloxyethanol to obtain 2-cyclohexyloxyethyl acetoacetate, BP 115-125°C/0.5mm (flash distillation).

The compounds of this invention may be produced in step-wise manner with isolation of intermediates, if desired, rather than by the preferred one pot technique discussed, supra. In all of these procedures the reactions involve a Michael addition -cyclic condensation of an oxoenamine or precursors thereof to a Knoevenagel condensation product. Thus, by selection of the proper reactants which are either commercially available or prepared by standard procedures, the following procedures are applicable and involve the separate preparation of the appropriately substituted amino crotonate, amino ketone or appropriately substituted benzylidene acetoacetic ester:

Amino Crotonate

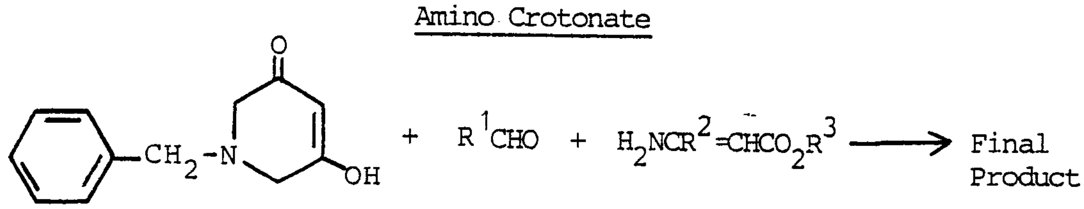


Amino Ketone

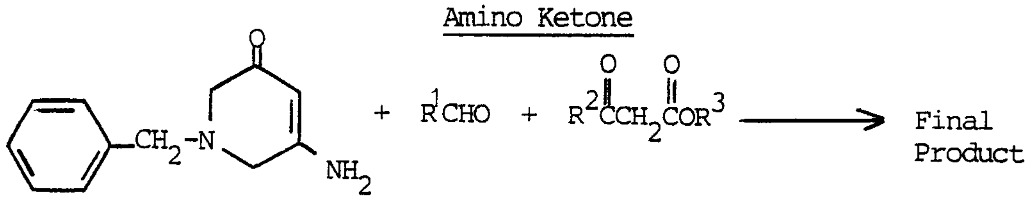


Benzylidene Acetoacetic Ester

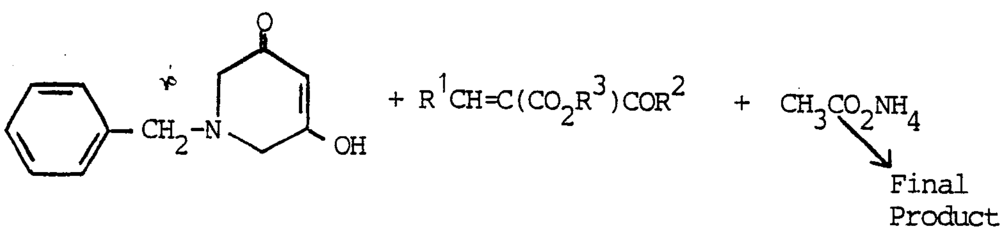


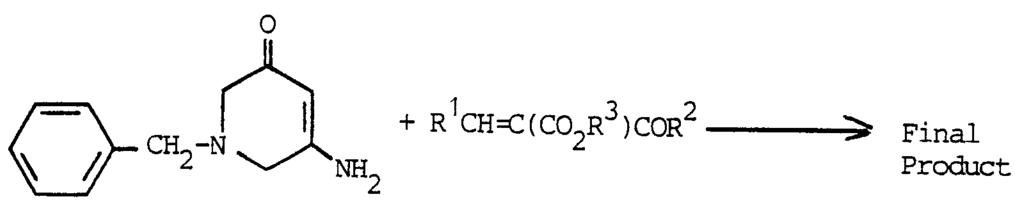


Benzylidene Diketone

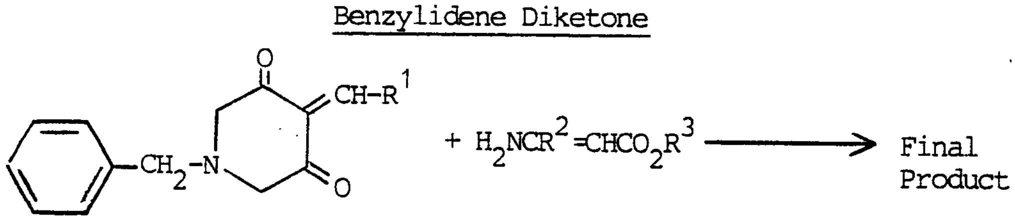

Thus, in a generic process sense the compounds of this invention can be produced by adding, with cyclic condensation (a Michael addition-cyclization), an appropriately substituted oxoenamine, or precursors thereof, to a Knoevenagel condensation product of an appropriately substituted aldehyde and an alkanoylacetic acid ester or a 1-benzyl-3-hydroxy-5-oxo-2,3,4,6-tetrahydropyridine. The N-benzyl protecting group is, of course, ultimately removed by hydrogenolysis.

The above mentioned reactions may be performed using the other N-protecting groups as defined above in place of benzyl. Similarly compounds of formula III may be produced using $R^{12}$ groups instead of $R^3$ in the starting materials. Compounds of formula II and processes for preparing them are also within the scope of this invention.

The pharmaceutically acceptable salts of the antihypertensive agents of this invention are prepared directly by neutralization of the free base or by metathetical displacement. The physiologically acceptable salts may be formed with organic or inorganic acids such as hydrochloride, hydrobromic, phosphoric, sulfuric, sulfonic, nitric, methylsulfonic, acetic, maleic, succinic, fumaric, tartaric, citric, salicyclic, lactic, naphthalene-sulfonic acid, and the like.

The compounds of formula I possess one or more asymmetric centres and hence optical isomers thereof are possible. All such isomers amd mixtures thereof are included within the scope of this invention. Where any reaction process produces mixtures of such isomers standard resolution techniques may be applied to separate a specific isomer, e.g. selective crystallisation or high performance liquid chromatography.

The compounds of this invention were initially shown to exhibit $Ca^{+2}$ antagonism in rabbit aortic smooth muscle following a modified procedure from that described by Brockaert et al., Eur.J.Pharmacol., 53, 281 (1979) whereby transverse strips (10mm $\times$ 2.5mm) from the thoracic aorta were cut and suspended vertically in a jacketed (37°C-50ml volume) organ bath in physiological saline solution (PSS) aerated with 95% $O_2$/5% $CO_2$. The composition of PSS was as follows (mM): NaCl 112,KCl 5, $NaHCO_3$ 25 $KH_2PO_4$ 1, $MgSO_4$ 1.2, $CaCl_2$ 2.5, dextrose 10. The lower end of each tissue strip was attached to a fixed post and the upper end to a Statham UC-4 transducer. Changes in force development were recorded on a Beckman Dynograph Polygraphic Recorder.

Following equilibration, the muscles were contracted in a depolarizing solution of PSS in which 100mM KCl was substituted for an equimolar concentration of NaCl. Following attainment of steady-state isometric force (20 min.), the test compound was added to afford a final concentration of 1 $\times$ $10^{-5}$M. The inhibitory effect, expressed as percent relaxation, was determined from the mean of two experiments twenty minutes after the addition of the compound being tested.

The potential for detrimental cardiac depressant (negative inotropic) effects of the compounds of this invention was assessed in isolated paced intact rabbit atria. Left and right atria (with nodal tissue excised) were suspended vertically in a jacketed (30°C) organ bath containing 50 ml of PSS and aerated with 95% $O_2$/5% $CO_2$. Muscles (N = 5-9/compound) were stimulated at a frequency of 3Hz with a WPI stimulator for a 60 minute equilibration period. Changes in isometric force were recorded as described for the aortic smooth muscle experiments, supra. Following equilibration, the test compound (or as a control, the ethanol vehicle) was added to the organ bath in a cumulative manner from doses ranging from $10^{-9}$M to $10^{-5}$M - ($10^{-5}$M was the maximum dose which could be attained due to the depressant effect of ethanol) and effects on developed isometric force were determined. Results are expressed as the concentration of calcium antagonist which produces 25% inhibition of isometric force ($IC_{25}$).

Known calcium entry blockers produced significant cardiac depression in this model. Verapamil ($IC_{25} = 6 \times 10^{-8}$M), nifedipine ($IC_{25} = 5 \times 10^{-8}$M), nitrendipine ($IC_{25} = 3.5 \times 10^{-7}$M) and felodipine - ($IC_{25} = 8 \times 10^{-7}$M) all produced direct negative inotropism. However, the known calmodulin inhibitor, W-7, produced less than 20% inhibition at the highest concentration ($10^{-5}$M) tested.

The hypotensive in vivo effect of the compounds of this invention was determined by measuring changes in the systolic blood pressure of spontaneously hypertensive rats with a Decker Caudal Plethysmograph. The compound being tested was administered to a group of 4 rats and their systolic pressure was determined prior to and at 1.5 and 4 hours after compound administration. Initial testing was done by oral administration of the compound. Results (mmHg) are expressed as decreases in systolic blood pressure.

The compounds were also shown, unlike nifedipine, verapamil and nitrendipine, and like the calmodulin antagonist W-7, to antagonise arterial actin-myosin interactions and $Ca^{+2}$-calmodulin dependent myosin light chain phosphorylation and subsequent contractile protein function when studied in accordance with the procedures of Silver et al., J.Pharmacol.Exp.Therap., 230, No.1, 141-148 - (1984). The data reported herein was obtained at a six minute time-point rather than the 1.5, 5 and 12 minute time points indicated in the superprecipitation assay of the Silver et al paper.

Thus, these data establish the compounds of this invention as $Ca^{+2}$ antagonists which are useful as antihypertensive agents functioning more at the vascular level than other known $Ca^{+2}$ entry blockers.

Based upon the activity profile elicited by the compounds of this invention in the above-described standard scientifically recognised test models, the compounds are established as hypotensive agents useful in the treatment of hypertension and conditions characterised by constrictive blood flow in coronary arteries. For that purpose, the compounds may be administered orally or parenterally in suitable dosage forms compatible with the route of administration, whether oral, intraperitoneal, intramuscular, intravenous, intranasal, buccal, etc. The effective dose range determined in the animal test models has been established at from 1 to about 50 milligrams per kilogram host body weight to be administered in single or plural doses as needed to obtain the desired hypotensive response. The specific dosage regimen for a given patient will depend upon age, pathological state, severity of dysfunction, size of the patient, etc. Oral administration is performed with either a liquid or solid dosage unit in any conventional form such as tablets, capsules, solutions, etc. which comprise a unit dose (e.g. from about 25 milligrams to about 4 grams) of the active ingredient alone or in combination with adjuvants needed for conventional coating, tableting, solubilizing, flavour or colouring. Parenteral administration with liquid unit dosage forms may be via sterile solutions or suspensions in aqueous or oleagenous medium. Isotonic aqueous vehicle for injection is preferred with or without stabilizers, preservatives and emulsifiers.

Thus the invention also provides a pharmaceutical composition comprising a compound having formula I or a pharmaceutically acceptable salt thereof in association with a pharmaceutically acceptable carrier. The composition may be prepared by bringing the compound having formula I or a pharmaceutically acceptable salt thereof into association with the said carrier, for example, by mixing the ingredients.

The following examples illustrate the preparation of a representative number of compounds of this invention. After each example, the $Ca^{+2}$ antagonist activity of the compound is presented in terms of percent relaxation of aortic tissue (P.R.) at $10^{-5}$M concentration. Also, the $IC_{25}$ data, where determined in atrial tissue, is presented for comparison purposes with that of standard $Ca^{+2}$ antagonists noted, supra, and to show that the compounds of this invention are operating more through the vascular smooth muscle regulatory mechanism than the known $Ca^{+2}$ entry blockers. Inhibition of superprecipitation (Antag. of Actin-Myosin interaction) is expressed as percent inhibition (mean ± standard error of the mean for at least 3 separate experiments) at a compound concentration. In the same manner, inhibition of myosin light chain phosphorylation (Antag. of MLCP) is expressed as a percent inhibition at a compound concentration. Similarly, the antihypertensive activity is reported in terms of millimetres mercury (mmHg) systolic blood pressure (B.P.) reduction at the stated time post 50 mg/kg oral dosing or other dosing as indicated.

## EXAMPLE 1

4-(2,3-Dichlorophenyl)-1,4,5,6,7,8-hexahydro-2-methyl-5-oxo-1,7-naphthyridine-3-carboxylic acid 2-methoxy-2-phenylethyl ester

a) A mixture of 15.2g of 1-benzyl-3-hydroxy-5-oxo-2,3,4,6-tetrahydropyridine hydrate, 17.7g of 2-methoxy-2-phenylethyl acetoacetate, 13.1g of 2,3-dichlorobenzaldehyde, 11.5g of ammonium acetate and 300 ml of methanol was refluxed for 6 hours. The solvent was removed in vacuo and the residue was slurried with ethyl acetate and filtered. The solid was dissolved in methylenechloride, extracted with water, then dried over magnesium sulfate. The methylene chloride was evaporated and the residue was slurried with diethyl ether and filtered to obtain 10.2g of solid, m.p. 220-2°C. Conversion to the hydrochloride afforded 10.3g of 4-(2,3-dichlorophenyl)-1,4,5,6,7,8-hexahydro-2-methyl-5-oxo-7-(phenylmethyl)-1,7-naphthyridine-3-carboxylic acid 2-methoxy-2-phenylethyl ester hydrochloride, m.p. 217°C dec.

| | |
|---|---|
| Analysis for: | $C_{32}H_{30}N_2Cl_2O_4 \cdot HCl$ |
| Calculated: | C, 62.60; H, 5.09; N, 4.56; Cl, 17.33 |
| Found: | C, 62.67; H, 5.10; N, 4.54; Cl, 17.66. |

(b) The above hydrochloride (7.8g), 200 ml of methanol, 10 ml of concentrated hydrochloric acid, and 0.5g of 10% palladium on charcoal were shaken with hydrogen (40 psig initial pressure) for 6 hours. The catalyst was separated and solvent was evaporated in vacuo. Ethanol was added to the residue and re-evaporated (3 times). The residue was slurried with diethyl ether and filtered. The solid was recrystallized from isopropanoldiethyl ether to obtain 4.7 of the title compound as the hydrochloride, m.p. 150-3°C.

| | |
|---|---|
| Analysis for: | $C_{25}H_{24}H_2Cl_2O_4$.HCl |
| Calculated: | C, 57.32; H, 4.81; N, 5.35; Cl, 20.30 |
| Found: | C, 57.49; H, 4.92; N, 5.68; Cl, 19.63 |

P.R. = 32

$IC_{25}$ = 1 × $10^{-5}$M

Antag. of Actin-Myosin -100µM = 83± 3%; 50µM = 75±3%; 25µM = 43± 2%

Antag. of MLCP -100µM = 76± 2%; 50µM = 66%; 25µM = 38± 3%

B.P.-dose 50mg/kg = -53mm at 1.5 hours; -30mm at 4 hours.

EXAMPLE 2

1,4,5,6,7,8-Hexahydro-2-methyl-5-oxo-4-(pentafluorophenyl)-1,7-naphthyridine-3-carboxylic acid 2-phenoxyethyl ester

a) Methanol (300 ml), 15.2g of 1-benzyl-3-hydroxy-5-oxo-2,3,4,6-tetrahydropyridine hydrate, 16.6g of phenoxyethyl acetoacetate, 14.7g of pentafluorobenzaldehyde and 11.5g of ammonium acetate were mixed and refluxed for 6 hours. A precipitate formed during this period. After cooling to room temperature, the mixture was filtered to obtain 20g of solid, m.p. 237-9°C dec. Conversion to the hydrochloride afforded 18.5g of 1,4,5,6,7,8-hexahydro-2-methyl-5-oxo-4-(pentafluorophenyl)-7-(phenylmethyl)-1,7-naphthyridine-3-carboxylic acid 2-phenoxyethyl ester hydrochloride, m.p. 208-210°C.

| | |
|---|---|
| Analysis for: | $C_{31}H_{25}N_2F_5O_4$.HCl |
| Calculated: | C, 59.95; H, 4.22; N, 4.51; Cl, 5.71 |
| Found: | C, 60.16; H, 4.38; N, 4.82; Cl, 5.42 |

b) A mixture of 16.4g of the above hydrochloride, 200 ml of methanol, 5 ml of concentrated hydrochloric acid, 15 ml of water and 0.5g of 10% palladium on carbon was shaken with hydrogen at an initial pressure of 50 psig. After shaking 18 hours, the catalyst was separated and the solution was evaporated to dryness in vacuo. The residue was slurried with ethanol and re-evaporated (3 times). The solid was separated and dissolved in boiling ethanol. The solution was evaporated to a small volume and diluted with diethyl ether. The precipitate was separated and dried to obtain 8.7g of the title compound as the hydrochloride salt, m.p. 230°C dec.

| | |
|---|---|
| Analysis for: | $C_{24}H_{19}N_2F_5O_4$.HCl |
| Calculated: | C, 54.30; H, 3.80; N, 5.28; Cl, 6.68 |
| Found: | C, 54.05; H, 3.82; N, 5.19; Cl, 6.71. |

P.R. = 71

$IC_{25}$ = 1 × $10^{-6}$M

Antag. of Actin-Myosin -100µM = 72± 2%; 50µM = 58± 4%; 25µM = 28± 2%

Antag. of MLCP -100µM = 69± 3%; 50µM = 54± 4%; 25µM = 24±3%

B.P. -dose 10 mg/kg = -39mm at 1.5 hours; -51mm at 4 hours.

EXAMPLE 3

(-)-1,4,5,6,7,8-Hexahydro-2-methyl-5-oxo-4-(pentafluorophenyl)-1,7-naphthridine-3-carboxylic acid 2-phenoxyethyl ester

1,4,5,6,7,8-Hexahydro-2-methyl-5-oxo-4-(pentafluorophenyl)-1,7-naphthyridine-3-carboxylic acid 2-phenoxyethyl ester (30.3g), 14.4g of (+) 2'-methyltartranilic acid and 1 litre of acetonitrile were heated to solution then left at room temperature for 18 hours. The precipitate was separated and the filtrate was set aside for later work-up. The solid was recrystallized twice from acetonitrile to obtain 11.5g of 2'-methyltartranilic acid salt, $[\alpha]^{26}_{D}$ = -85.24 [0.725; MeOH]. The salt was converted to 7.4g of base $[\alpha]^{26}_{D}$ = -181.96° [0.51; MeOH]. The base was suspended in methanol and treated with hydrogen chloride. The solution was evaporated to dryness in vacuo and the residue was dissolved in ethanol and re-evaporated (2 times). The residue was triturated with diethyl ether and filtered to obtain 7.3g of the title compound as the hydrochloride, $[\alpha]^{26}_{D}$ = -155.89° [0.705; MeOH].

| | |
|---|---|
| Analysis for: | $C_{24}H_{19}N_2F_5O_4$.HCl |
| Calculated: | C,54.30; H,3.80; N,5.28; Cl,6.68 |
| Found: | C,54.25; H,3.73; N,5.14; Cl,6.51. |

P.R. = 56

$IC_{25}$>1 × $10^{-5}$M

Antag. of Actin-Myosin -100µM = 76%; 50µM = 65%

Antag. of MLCP -100µM = 70%; 50µM = 57%

B.P. -dose 25 mg/kg = -6mm at 1.5 hours; -14mm at 4 hours.

EXAMPLE 4

(+)-1,4,5,6,7,8-Hexahydro-2-methyl-5-oxo-4-(pentafluorophenyl)-1,7-naphthyridine-3-carboxylic acid 2-phenoxyethyl ester

The original filtrate from Example 3 was evaporated to dryness and the residue was converted to 18.2g of base by treatment with saturated sodium carbonate solution. The recovered base and 8.7g of (-)-2'-methyltartranilic acid were dissolved in 370 ml of boiling acetonitrile. After standing at room temperature for 18 hours, the precipitate was separated and recrystallized twice from acetonitrile to obtain 13.5g of 2'-methyltartranilic acid salt, $[\alpha]^{25}_{D}$ = +87.33° [0.656; MeOH]. The salt was converted to 8.9g of base, $[\alpha]^{26}_{D}$ = +182,04° [0.635; MeOH]. The base was converted to the hydrochloride as in the previous example to obtain 8.5g of the title compound as the hydrochloride, $[\alpha]^{26}_{D}$ = +158.71° [0.62; MeOH].

| | |
|---|---|
| Analysis for: | $C_{24}H_{19}N_2F_5O_4$.HCl |
| Calculated: | C,54.30; H,3.80; N,5.28; Cl,6.68 |
| Found: | C,54.08; H,3.79; N,5.22; Cl,6.56. |

P.R. = 61

$IC_{25} = 8 \times 10^{-7}M$

Antag. of Actin-Myosin -100µM = 71%; 50µM = 38%

Antag. of MLCP -100µM = 67%; 50µM = 37%

B.P. -dose mg/kg = -58mm at 1.5 hours; -78mm at 4 hours.

EXAMPLE 5

1,4,5,6,7,8-Hexahydro-2-methyl-5-oxo-4-(pentafluorophenyl)-1,7-naphthyridine-3-carboxylic acid 2-methoxy-2-phenylethyl ester

a) A solution of 15.5g of 1-benzyl-3-hydroxy-5-oxo-2,3,4,6-tetrahydropyridine hydrate, 14.7g of pentafluorobenzaldehyde, 17.7g of 2-methoxy-2-phenylethyl acetoacetate, 11.6g of ammonium acetate and 300ml of methanol was heated at reflux for 8 hours. After cooling, the mixture was filtered to obtain 22.5 of solid. Recrystallization from ethanol afforded 17.3g of 1,4,5,6,7,8-hexahydro-2-methyl-5-oxo-4-(pentafluorophenyl)-7-(phenylmethyl)-1,7-naphthyridine-3-carboxylic acid 2-methoxy-2-phenylethyl ester, m.p. 220-2°C.

| | |
|---|---|
| Analysis for: | $C_{32}H_{27}N_2F_5O_4$ |
| Calculated: | C,64.20; H, 4.55; N, 4.68 |
| Found: | C,63.98; H, 4.49; N, 4.67. |

b) A mixture of 16.9g of the above solid, 250 ml of methanol, 2 ml of concentrated hydrochloric acid and 1g of 10% palladium on carbon was shaken with hydrogen (initial pressure 40 psig) for 13 hours. The catalyst was separated and the solution was evaporated to dryness in vacuo. The residue was treated with methylene chloride and saturated sodium bicarbonate solution. The methylenechloride solution was dried over magnesium sulfate, then evaporated to dryness. The residue was dissolved in 30 ml of ethyl acetate, filtered, and diluted with 30 ml of pentane. Filtration afforded 8.3g of solid, mp. 175-180°C. An ethanol solution of 2.3g of this solid was treated with an excess of ethanolic hydrogen chloride. The solution was evaporated to dryness. The residue was re-precipitated from ethyl acetate-pentane to obtain 2g of the title compound as the hydrochloride, hemi-hydrate, mp. 180-5°C dec.

| | |
|---|---|
| Analysis for: | $C_{25}H_{21}N_2F_5O_4.HCl.½H_2O$ |
| Calculated: | C,54.20;H,4.20;N,5.06;Cl,6.40;$H_2O$,1.60 |
| Found: | C,54.29;H,4.00;N,5.02;Cl,6.53;$H_2O$,1.96. |

P.R. = 71

$IC_{25} = 1 \times 10^{-6}M$

Antag. of Actin-Myosin -100µM = 75±4%; 50µM = 50±5%; 25µM = 20±5%

Antag. of MLCP -100µM = 70± 3%; 50µM = 48±2%; 25µM = 21±3%

B.P.-dose 25 mg/kg = -60mm at 1.5 hours; -72mm at 4 hours;
10 mg/kg = -41mm at 1.5 hours; -68mm at 4 hours;
5 mg/kg = -44mm at 1.5 hours; -38mm at 4 hours.

EXAMPLE 6

1,4,5,6,7,8,-Hexahydro-2-methyl-5-oxo-4-(pentafluorophenyl)-1,7-naphthyridine-3-carboxylic acid (R)-2-methoxy-2-phenylethyl ester

a) A mixture of 23g of 1-benzyl-3-hydroxy-5-oxo-2,3,4,6-tetrahydropyridine hydrate, 21.9g of pentafluorobenzaldehyde, 26.3g of R(-)-2-methoxy-2-phenylethyl acetoacetate, 17.3g of ammonium acetate and 200 ml of methanol was heated to reflux. Solid began to precipitate within 20 minutes. After five hours, the mixture was cooled and filtered. The solid was recrystallized from ethanol to obtain 25 g of material, mp. 221-2°C, $[\alpha]^{26}_{D}$ = 10.27 [0.935; MeOH]. The base was converted to the hydrochloride and recrystallized from acetonitrile to obtain 17.5g of 1,4,5,6,7,8-hexahydro-2-methyl-5-oxo-4-(pentafluorophenyl)-7-(phenylmethyl)-1,7-naphthridine-3-carboxylic acid -(R)-2-methoxy-2-phenylethyl ester hydrochloride, mp. 210-4°C dec; $[\alpha]^{25}_{D}$ = -18.24° [1.025;MeOH].

| | |
|---|---|
| Analysis for: | $C_{32}H_{27}N_2F_5O_4$.HCl |
| Calculated: | C,60.52; H,4.44;N,4.41;Cl,5.58 |
| Found: | C,60.73;H,4.46;N,4.51;Cl,5.38. |

b) Fourteen grams of the above solid, 200 ml of methanol, 7ml of concentrated hydrochloric acid, 10ml of water and 1g of 10% palladium on carbon were shaken with hydrogen at an initial pressure of 50 psig. After 4 hours, the catalyst was separated and the solution was evaporated to dryness in vacuo. The residue was stirred with ethyl acetate and saturated sodium carbonate solution. The ethyl acetate portion was dried over magnesium sulfate and evaporated to dryness. The residue was crystallized from 50ml of diethyl ether to obtain 8.5g of solid, mp. 159-161°C;$[\alpha]^{25}_{D}$ = -19.44° [1.065; MeOH]. The solid was dissolved in ethanol and saturated with hydrogen chloride. The solution was treated with charcoal, filtered, and evaporated to dryness. The residue was reprecipitated from methylene chloride-hexane to obtain 6.8g of the title compound as the hydrochloride, $[\alpha]^{25}_{D}$ = -21.16[1.12;MeOH].

| | |
|---|---|
| Analysis for: | $C_{25}H_{21}N_2F_5O_4$.HCl |
| Calculated: | C,55.10;H,4.07;N,5.14;Cl,6.51 |
| Found: | C,54.92;H,4.20;N,4.99;Cl,6.11. |

P.R. = 71

$IC_{25}$ = 6 × $10^{-7}$M

Antag. of Actin-Myosin -100µM = 74%; 50µM =64%

Antag. of MLCP -100µM = 70%; 50µM = 56%

B.P. -dose 10mg/kg = -56mm at 1.5 hours; -49mm at 4 hours.

EXAMPLE 7

1,4,5,6,7,8-Hexahydro-2-methyl-5-oxo-4-(pentafluorophenyl)-1,7-naphthyridine-3-carboxylic acid (S)-2-methoxy-2-phenylethyl ester

a) A mixture of 23 grams of 1-benzyl-3-hydroxy-5-oxo-2,3,4,6-tetrahydropyridine hydrate, 21.9g of pentafluorobenzaldehyde, 26.3g of S(+)-2-methoxy-2-phenylethyl acetoacetate, 17.3g of ammonium acetate and 200 ml of methanol was heated to reflux. Solid began to precipitate within 20 minuts. After 4 hours, the mixture was cooled and filtered. The precipitate was recrystallized from ethanol to obtain 22g of solid, m.p. 218-221°C;$[\alpha]^{25.5}_{D}$ = +10.14°[1.065; $CHCl_3$]. The base was converted to the hydrochloride and recrystallized from acetonitrile to obtain 15.5g of 1,4,5,6,7,8-hexahydro-2-methyl-5-oxo-4-(pentafluorophenyl)-7-(phenylmethyl)-1,7-naphthyridine-3-carboxylic acid (S)-3-methoxy-2-phenylethyl ester hydrochloride,mp. 210-4°C dec.; $[\alpha]^{25}_{D}$ = +20.94-[1.27;MeOH].

| | |
|---|---|
| Analysis for: | $C_{32}H_{27}N_2F_5O_4$.HCl |
| Calculated: | C,60.52;H,4.44;N,4.41;Cl,5.58 |
| Found: | C,60.80;H,4.41;N,4.37;Cl,5.47. |

b) Thirteen grams of the above hydrochloride, 200ml of methanol, 7ml of concentrated hydrochloric acid, 10ml of water and 1g of 10% palladium on carbon were shaken with hydrogen at an initial pressure of 50psig. After 4 hours, the catalyst was separated and the solution was evaporated to dryness in vacuo. The residue was dissolved in ethyl acetate and shaken with saturated sodium carbonate solution. The ethyl acetate portion was dried over magnesium sulfate, then evaporated to dryness. The residue was crystallized from 50ml of diethyl ether to obtain 6.2g of solid, mp. 158-160°C;$[\alpha]_D^{25}$= +20.10°[1.025;MeOH]. The solid was dissolved in ethanol and saturated with hydrogen chloride. The solution was treated with charcoal, filtered and evaporated to dryness. The residue was reprecipitated twice from methylenechloride-hexane to obtain 4.5g of the title compound as the hydrochloride, mono-hydrate, $[\alpha]_D^{26}$= +21.71° [1.055; MeOH].

| | |
|---|---|
| Analysis for: | $C_{25}H_{21}N_2F_5O_4$.HCl.$H_2O$ |
| Calculated: | C,53.34;H,4.30;N,4.98;Cl6.30 |
| Found: | C,53.37;H,4.13;N,4.95;Cl,6.23. |

P.R. = 68

$IC_{25}$ = 6.5 × $10^{-6}$M

Antag.of Actin-Myosin -100µM = 80%; 50µM = 67%

Antag.of MLCP -100µM = 74%; 50µM = 59%

B.P.-dose 10mg/kg=-51mm at 1.5hours;-54mm at 4 hours.

EXAMPLE 8

1,4,5,6,7,8-Hexahydro-2-methyl-5-oxo-4-(R or S-pentafluorophenyl)-1,7-naphthyridine-3-carboxylic acid(S)-2-methoxy-2-phenylethyl esters

a) Two grams of the product of Example 7 were separated into 2 diastereomers by preparative high performance liquid chromatography on a Waters Prep Pak C18 column using methanol and ammonium phosphate buffer. Diastereomer A - (494mgs) was obtained as the hydrochloride, three quarter hydrate. $[\alpha]_D^{25.4}$ = +66.70° [1.00; MeOH]; Isomeric purity by HPLC, 85.4% diastereomer A and 14.6% diastereomer B.

| | |
|---|---|
| Analysis for: | $C_{25}H_{21}N_2F_5O_4$.HCl.¾$H_2O$ |
| Calculated: | C,53.77;H,4.24;N,5.03;Cl,6.35 |
| Found: | C,53.77;H,3.98;N,5.03;Cl,6.23. |

P.R. = 50 (Note: Muscles were pre-treated.)

Antag. of Actin-Myosin -100µM = 69%; 50µM = 28%

Antag. of MLCP -100µM = 63%; 50µM = 28%

B.P.-dose 50mg/kg =-73mm at 1.5hours;-86mm at 4 hours.

b) Diastereomer B (655mgs) was isolated as the hydrochloride, hemihydrate, $[\alpha]_D^{25.4}$ =15.69° - [1.045; MeOH]; Isomeric purity by HPLC, 88.7% diastereomer B and 11.3% diastereomer A.

| | |
|---|---|
| Analysis for: | $C_{25}H_{21}N_2F_5O_4 \cdot HCl \cdot \frac{1}{2}H_2O$ |
| Calculated: | C,54.20;H,4.19;N,5.06;Cl,6.40 |
| Found: | C,554.08;H,3.99;N,5.06;Cl,6.02 |

P.R. = 53 (Note: Muscles were pre-treated.)

Antag. of Actin-Myosin -100µM = 64%; 50µM = 30%

Antag. of MLCP = 100µM = 62%; 50µM = 34%

B.P.-dose 50 mg/kg=-28mm at 1.5hours;-67mm at 4 hours.

EXAMPLE 9

4-(2-Chloro-6-fluorophenyl)-1,4,5,6,7,8-hexahydro-2-methyl-5-oxo-1,7-naphthyridine-3-carboxylic acid 2-methoxy-2-phenylethyl ester

a) A mixture of 15.2g of 1-benzyl-3-hydroxy-5-oxo-2,3,4,6-tetrahydropyridine hydrate, 17.7g of 2-methoxy-2-phenylethyl acetoacetate, 11.9 of 2-chloro-6-fluorobenzaldehyde, 11.5g of ammonium acetate and 300ml of methanol was refluxed for 6 hours. The solvent was removed in vacuo. The residue was dissolved in methylenechloride, washed with water, then dried over magnesium sulfate. The solvent was evaporated in vacuo and the residue was crystallized by trituration with ethyl acetate to obtain 13.7g of solid, mp. 160-4°C. Conversion to the hydrochloride afforded 13.6g of 4-(2-chloro-6-fluorophenyl)-1,4,5,6,7,8-hexahydro-2-methyl-5-oxo-7-(phenylmethyl)-1,7-naphthyridine-3-carboxylicacid 2-methoxy-2-phenylethyl ester hydrochloride,m.p.191-4°C.

| | |
|---|---|
| Analysis for: | $C_{32}H_{30}N_2ClFO_4 \cdot HCl$ |
| Calculated: | C,64.32;H,5.23;N,4.69;Cl,11.87 |
| Found: | C,64.17;H,5.20;N,4.94;Cl,11.58. |

b) A mixture of the above solid (11.6g), 2ml of concentrated hydrochloric acid, 10ml of water, 200ml of methanol and 0.5g of 10% palladium on carbon were shaken with hydrogen (40 psig initial pressure) for 3 hours. The catalyst was separated and the solution was evaporated to dryness. The residue was dissolved in ethyl acetate and left at room temperature for 3 days. The precipitate was separated and dissolved in 450ml of refluxing ethanol. The solution was evaporated to one-third volume and diluted with an equal volume of diethyl ether. After standing overnight, the solid was separated and dried to obtain 6.6g of the title compound as the hydrochloride, mp. 224°C dec.

| | |
|---|---|
| Analysis for: | $C_{25}H_{24}N_2ClFO_4 \cdot HCl$ |
| Calculated: | C,59.18;H,4.97;N,5.52;Cl,13.98 |
| Found: | C,59.21;H,4.94;N,5.54;Cl,13.69 |

P.R. = 28

Antag. of Actin-Myosin -100µM = 40%

Antag. of MLCP -100µM = 44%

B.P. -dose 50mg/kg = -37mm at 1.5hours;-14mm at 4 hours.

EXAMPLE 10

4-(3-chloro-2-methylphenyl)-1,4,5,6,7,8-hexahydro-2-methyl-5-oxo-1,7-naphthyridine-3-carboxylic acid 2-methoxy-2-phenylethyl ester

a) Methanol (200ml), 15.2g of 1-benzyl-3-hydroxy-5-oxo-2,3,4,6-tetrahydropyridine hydrate, 17.7g of 2-methoxy-2-phenylethyl acetoacetate, 11.6g of 3-chloro-2-methylbenzaldehyde and 11.5g of ammonium acetate were combined and refluxed for 6 hours. The methanol was evaporated and the residue was dissolved in methylenechloride. The solution was extracted with water, dried over magnesium sulfate, then evaporated to dryness. The viscous residue was dissolved in ethyl acetate and allowed to stand at room temperature for 3 days. The mixture was filtered to obtain 4.5g of solid, mp. 180-2°C. Conversion to the hydrochloride afforded 4.7g of 4-(3-chloro-2-methylphenyl)-1,4,5,6,7,8-hexahydro-2-methyl-5-oxo-7-(phenylmethyl)-1,7-naphthyridine-3-carboxylic acid 2-methoxy-2-phenylethyl ester hydrochloride.

b) The hydrochloride from step (a) above was mixed with 200 ml of methanol, 5 ml of concentrated hydrochloric acid, 15 ml of water and 0.5g of 10% palladium on carbon, then shaken with hydrogen (50 psig initial pressure) for 6 hours. The catalyst was separated and the solution was evaporated to dryness in vacuo. The residue was shaken with ethyl acetate and saturated sodium carbonate solution. The ethyl acetate portion was dried over magnesium sulfate, evaporated to a small volume, and left at room temperature for 3 days. The precipitate was separated, suspended in ethyl acetate and treated with hydrogen chloride until solution occurred. Precipitate formed on scratching the sides of the flask. The solid was separated and dried to obtain 2.4g of the title compound as the hydrochloride, hemihydrate, mp. 160-3°C.

| | |
|---|---|
| Analysis for: | $C_{26}H_{27}N_2Cl_4.HCl.\frac{1}{2}H_2O$ |
| Calculated: | C,60.94;H,5.70;N,5.47;Cl,13.84 |
| Found: | C,60.93;H,5.60;N,5.48;Cl,14.16. |

P.R. = 60

$IC_{25} > 1 \times 10^{-5}$M

Antag. of Actin-Myosin -100µM = 76±2%; 50µM = 68±2%; 25µM = 45±3%

Antag.of MLCP -100µM = 75±3%; 50µM = 62±1%; 25µM = 35±5%

B.P.-dose 10mg/kg = -28mm at 1.5 hours;-19mm at 4 hours.

EXAMPLE 11

4-(3-Chlorophenyl)-1,4,5,6,7,8-hexahydro-2-methyl-5-oxo-1,7--naphthyridine-3-carboxylic acid 2-methoxy-2-phenylethyl ester

a) A mixture of 15.3g of 1-benzyl-3-hydroxy-5-oxo-2,3,4,6-tetrahydropyridine hydrate, 10.6g of 3-chlorobenzaldehyde, 17.7g of 2-methoxy-2-phenylethyl acetoacetate, 12g of ammonium acetate and 150ml of ethanol was refluxed for 5 hours. The solvent was removed in vacuo. The residue was dissolved in methylenechloride, extracted with water, then dried over magnesium sulfate. The solution was diluted with diethyl ether to the point of cloudiness, then left at room temperature for 18 hours. The supernatant was decanted and the precipitate was slurried with ethanol and filtered to obtain 12.9g of the solid, mp.168-170°C dec. The solid was suspended in methanol and saturated with hydrogen chloride. The mixture was cooled in an ice bath, then filtered. The solid was recrystallized from ethanol to obtain 11.1g of 4-(3-chlorophenyl)-1,4,5,6,7,8-hexahydro-2-methyl-5-oxo-7-(phenylmethyl)-1,7-naphthyridine-3-carboxylic acid 2-methoxy-2-phenylethyl ester hydrochloride, mp. 222-4°C dec.

b)The hydrochloride from step (a) (10.8g), 1g of 10% palladium on carbon and 450ml of methanol were shaken with hydrogen at an initial pressure of 48.5 psig. After 4 hours, the catalyst was separated and the solution was evaporated to dryness. The residue was dissolved in methanol, saturated with hydrogen chloride, then evaporated to

dryness. The residue was recrystallized from ethanol to obtain 3g of the title compound as the hydrochloride, monohydrate, mp. 165-7°C.

| | |
|---|---|
| Analysis for: | $C_{25}H_{25}N_2ClO_4 \cdot HCl \cdot H_2O$ |
| Calculated: | C,59.17;H,5.56;N,5.52;Cl,13.97 |
| Found: | C,59.02;H,5.62;N,5.70;Cl,13.51. |

P.R. = 31

Antag. of Actin-Myosin -100µM = 53%

Antag. of MLCP -100µM = 48%

B.P.-dose 50mg/kg=-29mm at 1.5 hours; -38mmm at 4 hours.

EXAMPLE 12

1,4,5,6,7,8-Hexahydro-2-methyl-5-oxo-4-[2-(trifluoromethyl)phenyl]-1,7-naphthyridine-3-carboxylic acid 2-methoxy-2-phenylethyl ester

a) In a manner similar to Example 11(a) o-trifluoromethylbenzaldehyde, 1-benzyl-3-hydroxy-5-oxo-2,3,4,6-tetrahydropyridine, 2-methoxy-2-phenylethyl acetoacetate and ammonium acetate were reacted to obtain 1,4,5,6,7,8-hexahydro-2-methyl-5-oxo-7-(phenylmethyl)-4-[2-(trifluoromethyl)phenyl]-1,7-naphthyridine-3-carboxylic acid 2-methoxy-2-phenylethyl ester, isolated as the hydrochloride, m.p. 175-8°C dec.

| | |
|---|---|
| Analysis for: | $C_{33}H_{31}N_2F_3O_4 \cdot HCl$ |
| Calculated: | C,64.65;H,5.26;N,4.57;Cl,5.78 |
| Found: | C,64.94;H,5.15;N,4.47;Cl,5.95. |

b) Hydrogenation of the above material afforded the title compound as the hydrochloride, m.p. 179-181°C.

| | |
|---|---|
| Analysis for: | $C_{26}H_{25}N_2F_3O_4 \cdot HCl$ |
| Calculated: | C,59.71;H,5.01;N,5.36;Cl,6.78 |
| Found: | C,59.83;H,5.09;N,5.54;Cl,7.18. |

P.R. = 39

$IC_{25}$ = 2 × $10^{-6}$M

Antag.of Actin-Myosin -100µM = 30%

Antag.of MLCP -100µM = 24%

B.P. -dose 25mg/kg =-56mm at 1.5hours; -56mm at 4hours.

EXAMPLE 13

1,4,5,6,7,8-Hexahydro-2-methyl-5-oxo-4-(pentafluorophenyl)-1,7-naphthyridine-3-carboxylic acid 1-methyl-2-phenoxyethyl ester

a) A mixture of 15.2g of 1-benzyl-3-hydroxy-5-oxo-2,3,4,6-tetrahydropyridine hydrate, 17.7g of 1-methyl-2-(phenoxy)ethyl acetoacetate, 14.7g of pentafluorobenzaldehyde, 11.5g ammonium acetate and 300ml of methanol was heated to reflux for 6 hours. After evaporation of the methanol, the solid residue was dissolved in methylenechloride and extracted with water. The methylenechloride solution was dried over magnesium sulfate, then evaporated to dryness. The residue was recrystallized from ethyl acetate-hexane to obtain 14g of solid, m.p. 204-8°C. Conversion to the hydrochloride afforded 8.6g of 1,4,5,6,7,8-hexahydro-2-methyl-2-methyl-5-oxo-4-(pentafluorophenyl)-7-(phenylmethyl)-1,7-naphthyridine-3-carboxylic acid 1-methyl-2-(phenoxy)ethyl ester hydrochloride,m.p. 215-7°C.

| | |
|---|---|
| Analysis for: | $C_{32}H_{27}N_2F_5O_4$.HCl |
| Calculated: | C,60.52;H,4.44;N,4.41;Cl5.58 |
| Found: | C,60.80;H,4.47;N,4.45;Cl,5.54 |

b) The above hydrochloride (7.6g), 200ml of methanol, 5ml of concentrated hydrochloric acid, 15ml of water and 0.5g of 10% palladium on carbon were shaken with hydrogen (50 psig initial pressure) for 6 hours. The catalyst was removed and the solution was evaporated in vacuo. The residue was shaken with diethyl ether and saturated sodium carbonate solution. The ether solution was dried over magnesium sulfate, then evaporated to dryness. The solid residue was suspended in methanol and treated with hydrogen chloride until solution occurred. The solvent was removed in vacuo and the residue was left standing with ethyl acetate-hexane for 3 days. The solid was separated and dried to obtain 3.2g of the title compound as the hydrochloride, m.p. indefinite.

| | |
|---|---|
| Analysis for: | $C_{25}H_{21}N_2F_5O_4$.HCl |
| Calculated: | C,55.10;H,4.07;N,5.14;Cl,6.51 |
| Found: | C,54.62;H,4.06;N,5.02;Cl,6.55. |

P.R. = 46

$IC_{25}$ = 1 × $10^{-5}$M

Antag. of Actin-Myosin -100µM = 78%; 25µM = 61%

Antag. of MLCP -100µM = 70%; 25µM = 58%

B.P. -dose 50 mg/kg = -35mm at 1.5 hours; -60mm at 4 hours.

EXAMPLE 14

1,4,5,6,7,8-Hexahydro-2-methyl-5-oxo-4-(pentafluorophenyl)-1,7-naphthyridine-3-carboxylic acid 2,3-dihydro-1,4-benzodioxin-2-ylmethyl ester

a) Methanol (300ml), 15.2g of 1-benzyl-3-hydroxy-5-oxo-2,3,4,6-tetrahydropyridine hydrate, 18.8g of 2,3-dihydro-1,4-benzodioxin-2-ylmethyl acetoacetate, 14.7g pentafluorobenzaldehyde, and 11.5g of ammonium acetate were combined and heated at reflux for 6 hours. The mixture was cooled, then the precipitate was separated by filtration. The solid was dissolved in methylenechloride, extracted with water, then dried over magnesium sulfate and evaporated to dryness. The residue was slurried with diethyl ether and filtered to obtain 14.6g of solid, m.p. 215-220°C dec. Conversion to the hydrochloride afforded 15.2g of 1,4,5,6,7,8-hexahydro-2-methyl-5-oxo-4-(pentafluorophenyl)-7-(phenylmethyl)-1,7-naphthyridine-3-carboxylic acid (2,3-dihydro-1,4-benzodioxin-2-yl)methyl ester hydrochloride, m.p. 190-3°C.

| | |
|---|---|
| Analysis for: | $C_{32}H_{25}N_2F_5O_5$.HCL |
| Calculated: | C,59.22;H,4.04;N,4.31;Cl,5.46 |
| Found: | C,58.96;H,4.00;N,4.27;Cl,5.52 |

b) Twelve grams of the above hydrochloride, 200ml of methanol, 5ml of concentrated hydrochloric acid, 15 ml of water and 0.5g of 10% palladium on carbon were shaken with hydrogen (50 psig initial pressure) for 6 hours. After separation of the catalyst, the solution was evaporated to dryness in vacuo. The residue was dissolved in ethyl acetate and water. The ethyl acetate portion was dried over magnesium sulfate, then evaporated to dryness. The residue solidified on standing with ethanol-ether for 3 days. The solid was treated with boiling hexane, filtered and dried to obtain 4.2g of the title compound as the hydrochloride, m.p. 167-170°C.

| | |
|---|---|
| Analysis for: | $C_{25}H_{19}N_2F_5O_5$.HCl |
| Calculated: | C,53.72; H,3.61;N,5.01;Cl,6.34 |
| Found: | C,53.51;H,3.64;N,5.00;Cl,6.09. |

P.R. = 62

$IC_{25}$ = 3.2 × $10^{-6}$M

Antag.of Actin-Myosin -100µM = 80%; 25µM = 65%

Antag.of MLCP -100µM = 76%; 25µM = 59%

B.P. -dose 50 mg/kg =-46mm at 1.5 hours; -42mm at 4 hours.

EXAMPLE 15

1,4,5,6,7,8-Hexahydro-2-methyl-4-(2-methylphenyl)-5-oxo-1,7-naphthyridine-3-carboxylic acid 2-phenoxyethyl ester

a) A mixture of 15.2g of 1-benzyl-3-hydroxy-5-oxo-2,3,4,6-tetrahydropyridine hydrate, 16.6g of 2-phenoxyethyl acetoacetate, 9g of o-tolualdehyde, 11.5g of ammonium acetate and 300 ml of methanol was refluxed 6 hours. The solvent was evaporated to the point of crystallization, then cooled and filtered to obtain 22.7g of solid, m.p. 165-7°C. The solid was dissolved in methanol and saturated with hydrogen chloride. The precipitated solid was separated, washed with methanol and dried to obtain 23.5 of 1,4,5,6,7,8-hexahydro-2-methyl-4-(2-methylphenyl)-5-oxo-7-(phenylmethyl)-1,7-naphthyridine-3-carboxylic acid 2-phenoxyethyl ester hydrochloride, m.p. 219-222°C.

| | |
|---|---|
| Analysis for: | $C_{32}H_{32}N_2O_4$.HCl |
| Calculated: | C,70.51;H,6.10;N,5.14;Cl,6.50 |
| Found: | C,70.49;H,5.96;N,5.03;Cl,6.20. |

b) The hydrochloride from above (21.5g), 500ml of methanol, 5ml of concentrated hydrochloric acid, 15ml of water and 0.6g of 10%palladium on carbon were shaken with hydrogen (50 psig initial pressure) for 20 hours. The catalyst was separated and the solution was evaporated to dryness in vacuo. The residue was slurried with diethyl ether and filtered. The solid was triturated with refluxing ethanol, filtered and dried to obtain 12g of the title compound as the hydrochloride, m.p. 238-240°C dec.

| | |
|---|---|
| Analysis for: | $C_{25}H_{26}N_2O_4$.HCl |
| Calculated: | C,66.00;H,5.98;N,6.16;Cl,7.79 |
| Found: | C,66.28;H,5.91;N,6.31;Cl,8.09. |

P.R. = 63

$IC_{25}$ = 4.7 × $10^{-6}$M

Antag. of Actin-Myosin -100µM = 66±5%

Antag. of MLCP -100µM = ±5%

B.P.-dose 25mg/kg = -29mm at 1.5 hours;-39mm at 4 hours.

EXAMPLE 16

1,4,5,6,7,8-Hexahydro-2-methyl-5-oxo-4-(2,3,6-trifluorophenyl)-1,7-naphthyridine-3-carboxylic acid 2-phenoxyethyl ester

a) Eight grams of 2,3,6-trifluorobenzaldehyde, 10.2g of 1-benzyl-3-hydroxy-5-oxo-2,3,4,6-tetrahydropyridine hydrate, 11.5g of 2-phenoxyethyl acetoacetate, 7.7g of ammonium acetate and 125ml of methanol were combined and refluxed 6 hours. The mixture was evaporated to dryness in vacuo. The residue was dissolved in methylenechloride and extracted with water. The methylenechloride solution was evaporated and the residue was dissolved in ethanol and re-evaporated. The residue was triturated with ethanol until it solidified. The mixture was filtered to obtain 12g of solid, m.p. 168-9°C. The solid was suspended in methanol and treated with hydrogen chloride. The solution was evaporated and the residue was triturated with diethyl ether to obtain 12.5g of 1,4,5,6,7,8-hexahydro-2-methyl-5-oxo-7-(phenylmethyl)-4-(2,3,6-trifluorophenyl)-1,7-naphthyridine-3-carboxylic acid 2-phenoxyethyl ester hydrochloride.

b) The above solid, 200ml of methanol of water, 5ml of concentrated hydrochloric acid and 1g of 10% palladium on carbon were shaken with hydrogen at an initial pressure of 50 psig. After 5 hours, the catalyst was separated and the solution was evaporated to dryness. The residue was slurried with ethanol and filtered. The solid was recrystallized from methanol-diethyl ether to obtain 7g of the title compound as the hydrochloride, m.p. 252-5°C dec.

| | |
|---|---|
| Analysis for: | $C_{24}H_{21}N_2F_3O_4$.HCl |
| Calculated: | C,58.24;H,4.48;N,5.66;Cl,7.17 |
| Found: | C,58.22;H,4.34;N,5.68;Cl,7.27. |

P.R. = 63

Antag. of Actin-Myosin = 100µM = 34%

Antag. of MLCP -100µM = 28%

B.P. -dose 25 mg/kg = -37mm at 1.5 hours; -18mm at 4 hours.

EXAMPLE 17

4-(3-Chloro-2-methylphenyl)-1,4,5,6,7,8-hexahydro-2-methyl-5-oxo-1,7-naphthyridine-3-carboxylic acid 2-phenoxyethyl ester

a) A mixture of 14.2g of 1-benzyl-3-hydroxy-5-oxo-2,3,4,6-tetrahydropyridine hydrate, 10.8g of 3-chloro-2-methylbenzaldehyde, 15.6g of 2-phenoxyethyl acetoacetate, 10.8g of ammonium acetate and 300ml of methanol was heated at reflux for 6 hours. The solvent was evaporated and the residue was dissolved in methylene chloride. The methylenechloride solution was extracted with water, dried over magnesium sulfate, then evaporated to dryness. The residue was recrystallized from ethanol to obtain 6.4g of solid, m.p. 188-190°C. The solid was suspended in methanol and treated with hydrogen chloride to obtain 6.3g of 4-(3-chloro-2-methylphenyl)-1,4,5,6,7,8-hexahydro-2-methyl-5-oxo-7-(phenylmethyl)-1,7-naphthyridine-3-carboxylic acid 2-phenoxyethyl ester hydrochloride, m.p. 202-5°C.

(b) The solid form above, 200 ml of ethanol, 5 ml of concentrated hydrochloric acid, 15 ml of water and 0.5g of 10% palladium on carbon were shaken with hydrogen at an initial pressure of 50 psig. After 3 hours, the catalyst was separated and the solution was evaporated to dryness. The residue was dissolved in ethanol and evaporated to

dryness (3 times). The solid was recrystallized from ethanol to obtain 1.7g of the title compound as the hydrochloride, m.p. 233-5°C.

| | |
|---|---|
| Analysis for: | $C_{26}H_{25}N_2ClO_4.HCl$ |
| Calculated: | C,61.35;H,5.35;N,5.72;Cl,14.48 |
| Found: | C,61.17;H,5.31;N,5.69;Cl,14.14 |

P.R. = 74

$IC_{25}$ = 1 × $10^{-6}$M

Antag. of Actin-Myosin -100µM =71±2%; 50µM=64±3%; 25µM=44±2%; 10µM=22%

Antag.of MLCP-100µM=67±2%; 50µM=59±3%; 25µM=39±2%;10µM=11% B.P. -dose 25mg/kg=-10mm at 1.5hours;-45mm at 4 hours.

EXAMPLE 18

1,4,5,6,7,8-Hexahydro-2-methyl-5-oxo-4-(pentafluorophenyl)-1,7-naphthyridine-3-carboxylic acid 2-phenylethyl ester

(a) A mixture of 12g of 1-benzyl-3-hydroxy-5-oxo-2,3,4,6-tetrahydropyridine, 11.8g of pentafluorobenzaldehyde, 12.6g of phenylethyl acetoacetate, 14g of ammonium acetate and 200 ml of isopropanol was refluxed for 5 hours. The solution was evaporated to dryness in vacuo. The residue was slurried with 95% ethanol and filtered. The solid was washed with water and methanol, then dried to obtain 15.3g of material, m.p. 213-5°C. The solid was suspended in methanol and saturated with hydrogen chloride. The solution was evaporated to dryness. The residue was dissolved in ethanol and re-evaporated. The residue was slurried with 50:50 ethanol/diethyl ether and filtered to obtain 11.8g of 1,4,5,6,7,8-hexahydro-2-methyl-5-oxo-7-(phenylmethyl)-4-(pentafluorophenyl)-1,7-naphthyridine-3-carboxylic acid 2-phenylethyl ester hydrochloride,m.p. 213°C.

(b) The hydrochloride from step (a),450ml of methanol and 1g of 10% palladium on carbon were shaken with hydrogen (47.5psig initial pressure) for 3.5 hours. The catalyst was separated and the solution was evaporated to dryness in vacuo. The residue was dissolved in methylenechloride, dried over magnesium sulfate, then evaporated to dryness in vacuo. The solid residue was re-precipitated from methylenechloride-hexane to obtain the title compound as the hydrochloride, hemi-hydrate, m.p. indefinite.

| | |
|---|---|
| Analysis for: | $C_{24}H_{19}N_2F_5O_3.HCl.\frac{1}{2}H_2O$ |
| Calculated: | C,55.02;H,4.04;N,5.35;Cl,6.77 |
| Found: | C,55.19;H,3.91;N,5.43;Cl,7.11 |

P.R. = 48

$IC_{25}$ = 1 × $10^{-6}$M

Antag.of Actin-Myosin -100µM=78±2%; 50µM=65%; 25µM=42%

Antag.of MLCP -100 µM=77±3%; 50µM=64%; 25µM=35%

B.P. -dose 10mg/kg=-27mm at 1.5 hours;-24mm at 4 hours.

EXAMPLE 19

4-(2,3-Dichlorophenyl)-1,4,5,6,7,8-hexahydro-2-methyl-5-oxo-1,7-naphthyridine-3-carboxylic acid 2-phenoxyethyl ester

(a) Methanol (300ml), 16.6g of 1-benzyl-3-hydroxy-5-oxo-2,3,4,6-tetrahydropyridine hydrate, 13.2g of 2,3-dichlorobenzaldehyde, 16.7g of 2-phenoxyethyl acetoacetate, and 11.6g of ammonium acetate were combined and refluxed for 6 hours. The mixture was cooled and filtered to obtain 8.3g of solid, m.p. 186-8°C dec. Conversion to the hydrochloride afforded 8.2g of 4-(2,3-dichlorophenyl)-1,4,5,6,7,8-hexahydro-2-methyl-5-oxo-7-phenylmethyl-1,7-naphthyridine-3-carboxylic acid 2-phenoxyethyl ester hydrochloride, m.p. 208-210°C dec.

(b) The solid from step (a), 400ml of methanol, 3ml of concentrated hydrochloric acid and 0.5g of 10% palladium on carbon were shaken with hydrogen (48.5 psig initial pressure) for 18 hours. The catalyst was separated and the solution was evaporated to dryness. The residue was recrystallized three times from methanol to obtain the title compound as the hydrochloride, m.p. 228-230°C dec.

| | |
|---|---|
| Analysis for: | $C_{24}H_{22}N_2Cl_2O_4 \cdot HCl$ |
| Calculated: | C,56.53;H,4.55;N,5.50;Cl,20.86 |
| Found: | C,56.39;H,4.75;N,5.52;Cl,20.56 |

P.R. = 62

$IC_{25}$ = 2.5 × $10^{-6}$M

Antag.of Actin-Myosin -100µM = 71%

Antag.of MLCP -100µM = 69%

B.P.-dose 50mg/kg = -50mm at 1.5 hours;-73mm at 4 hours.

EXAMPLE 20

1,4,5,6,7,8-Hexahydro-2-methyl-5-oxo-4-(pentafluorophenyl)-1,7 -naphthyridine-3-carboxylic acid 2-methyl-2-phenoxypropyl ester

(a) A mixture of 11.8g of 1-benzyl-3-hydroxy-5-oxo-2,3,4,6-tetrahydropyridine hydrate, 13.3g of (2-methyl-2-phenoxy)propyl acetoacetate, 10.6g of pentafluorobenzaldehyde, 8.5g of ammonium acetate and 125ml of methanol were combined and refluxed for 4 hours. The mixture was cooled, dilutes with 125ml of 95% ethanol, then filtered to obtain 17g of solid, m.p. 190-3°C. The solid was suspended in methanol and treated with hydrogen chloride to afford 17g of 1,4,5,6,7,8-hexahydro-2-methyl-5-oxo-4-(pentafluorophenyl)-7-phenylmethyl-1,7-naphthyridine-3-carboxylic acid 2-methyl-2-phenoxypropyl ester hydrochloride, m.p. 176-9°C.

(b) The above hydrochloride (15g), 400ml of methanol, 10ml of water, 8ml of concentrated hydrochloric acid, and 1g of 10% palladium on carbon were shaken with hydrogen (50 psig initial pressure) for 4.5 hours. The catalyst was separated and the filtrate was evaporated to dryness in vacuo. The residue was dissolved in methylene chloride and shaken with saturated sodium carbonate solution. The methylene chloride solution was separated and a precipitate formed after 20 minutes. The mixture was filtered to obtain 6.5g of solid, m.p. 170-3°C. The solid was dissolved in methanol and treated with hydrogen chloride to obtain 4.9g of the title compound as the hydrochloride.

| | |
|---|---|
| Analysis for: | $C_{26}H_{23}F_5N_2O_4 \cdot HCl$ |
| Calculated: | C,55.89;H,4.33;N,5.01;Cl,6.34 |
| Found: | C,55.48;H,4.31;N,5.03;Cl,6.70 |

P.R. = 51

Antag. of Actin-Myosin -100µM = 67%

Antag. of MLCP -100µM = 62%

B.P. -dose 25 mg/kg = -64mm at 1.5 hours; -41mm at 4 hours.

EXAMPLE 21

1,4,5,6,7,8-Hexahydro-2-methyl-5-oxo-4-(pentafluorophenyl)-1,7-naphthyridine-3-carboxylic acid 4-phenoxybutyl ester

(a) Pentafluorobenzaldehyde (12.5g), 15.6g of 4-phenoxybutyl acetoacetate, 15g of 1-benzyl-3-hydroxy-5-oxo-2,3,4,6-tetrahydropyridine hydrate, 11g of ammonium acetate and 200 ml of methanol were combined and heated to reflux. A precipitate formed after one-half hour. After heating for 5.5 hours, the mixture was cooled and filtered to obtain 9.5g of 1,4,5,6,7,8-hexahydro-2-methyl-5-oxo-4-(pentafluoro)-7-(phenylmethyl)-1,7-naphthyridine-3-carboxylic acid 4-phenoxybutyl ester,m.p. 229-231°C.

(b) The solid from step (a) was dissolved in 200ml of methanol and saturated with hydrogen chloride. Water (10ml) and 0.6g of 10% palladium on carbon were added and the mixture was shaken with hydrogen (50 psig initial pressure) for 5 hours. The catalyst was separated and the solution was concentrated to dryness. The residue was dissolved in ethanol and evaporated to dryness. This was repeated until a solid residue was obtained. The residue was recrystallized from ethanol to obtain 4.8g of the title compound as the hydrochloride, m.p. 210°C dec.

| | |
|---|---|
| Analysis for: | $C_{26}H_{23}N_2F_5O_4$.HCl |
| Calculated: | C,55.87;H,4.33;N,5.01;Cl,6.34 |
| Found: | C,55.74;H,4.23;N,4.99;Cl,6.59 |

P.R. = 61

$IC_{25}$ = 1 × $10^{-6}$M

Antag. of Actin-Myosin -100μM = 80%

Antag. of MLCP -100μM = 77%

B.P.-dose 25mg/kg = 0mm at 1.5 hours;-49mm at 4 hours.

EXAMPLE 22

1,4,5,6,7,8-Hexahydro-2-methyl-5-oxo-4-(pentafluorophenyl)-1,7-naphthyridine-3-carboxylic acid 2-(2,3-dichlorphenoxy)ethyl ester

(a) Methanol (250 ml), 9.1g of 1-benzyl-3-hydroxy-5-oxo-2,3,4,6-tetrahydropyridine hydrate, 13.3g of 2-(2,3-dichlorophenoxy)ethyl acetoacetate, 8.8g of pentafluorobenzaldehyde, and 6.9g of ammonium acetate were combined and refluxed for 6 hours. The mixture was cooled and filtered. The solid was dissolved in ethyl acetate, extracted with water, then dried over magnesium sulfate. Evaporation of the solvent afforded 12g of solid, m.p. 223-6°C dec. The solid in methanol was treated with hydrogen chloride to obtain 12.4g of 1,4,5,6,7,8-hexahydro-2-methyl-5-oxo-7-(phenylmethyl)-4-(pentafluorophenyl)-1,7-naphthyridine-3-carboxylic acid 2-(2,3-dichlorophenoxy)ethyl ester hydrochloride, m.p. 223-6°C dec.

(b) The hydrochloride from above (10g), 500 ml of methanol, 25 ml of water, 5 ml of concentrated hydrochloric acid and 0.5g of 10% palladium on carbon were shaken with hydrogen (45 psig initial pressure) for 4 hours. The catalyst was separated and the solution was evaporated to dryness. The residue was dissolved in ethyl acetate and shaken with dilute sodium hydroxide solution. The ethyl acetate solution was dried over magnesium sulfate, then evaporated to dryness. The residue was dissolved in methanol and saturated with hydrogen chloride. The solution was evaporated to dryness. The residue crystallized on standing with ethyl acetate for 3 days. The solid was treated with boiling ethyl acetate and filtered to obtain the title compound as the hydrochloride, m.p. 228-231°C dec.

| | |
|---|---|
| Analysis for: | $C_{24}H_{17}N_2Cl_2F_5O_4$.HCl |
| Calculated: | C,48.06;H,3.02;N,4.67;Cl,17.74 |
| Found: | C,48.38;H,3.04;N,4.71;Cl,17.40 |

P.R. = 61

$IC_{25} = 1 \times 10^{6}M$

Antag. of Actin-Myosin - 100µM=78±1%;50µM=75±4%;25µM=54±3%; 10µM=30±3%

Antag.of MLCP-100µM=74±2%; 50µM=73±1.5%; 25µM=54±2.5%;10µM=25%

B.P.-dose 25mg/kg=-37mm at 1.5 hours;-52mm at 4 hours.

EXAMPLE 23

4-(2-chloro-6-fluoro-3-methylphenyl)-1,4,5,6,7,8-hexahydro-2-methyl-5-oxo-1,7-naphthyridine-3-carboxylic acid 2-phenoxyethyl ester

(a) Methanol (200ml), 16.6g of 1-benzyl-3-hydroxy-5-oxo-2,3,4,6-tetrahydropyridine hydrate, 16.6g of 2-phenoxyethyl acetoacetate, 13g of 2-chloro-6-fluoro-3-methylbenzaldehyde and 10g of ammonium acetate were combined and relfuxed for 5 hours. The solution was evaporated to dryness. The residue was dissolved in methylenechloride, extracted with water then dried over magnesium sulfate. The solvent was evaporated and the residue was left standing with diethyl ether for 18 hours. The ether was decanted from the solid residue. The residue was treated with hot methanol and filtered to obtain 22g of solid, m.p. 174-7°C. The solid was suspended in methanol and treated with hydrogen chloride. The solution was evaporated and the residue was dissolved in 100 ml of methanol. After 2 days at room temperature, the solid was separated and recrystallized from ethanol to obtain 13.5g of 4-(2-chloro-6-fluoro-3-methylphenyl)-1,4,5,6,7,8-hexahydro-2-methyl-5-oxo-7-(phenylmethyl)-1,7-naphthyridine-3-carboxylic acid 2-phenoxyethyl ester hydrochloride, m.p. 185-7°C.

(b) Twelve grams of the above hydrochloride, 125ml of methanol, 2 ml of concentrated hydrochloric acid, 10 ml of water, and 1 g of 10% palladium on carbon were shaken with hydrogen - (50 psig initial pressure) for 24 hours. The mixture was diluted with 500 ml of methanol, heated to reflux and filtered. The filtrate was evaporated to dryness and the residue was slurried with ethanol and filtered. The solid was recrystallized from methanol to obtain 5.2g of the title compound as the hydrochloride, m.p. 252-5°C dec.

Analysis for: $C_{25}H_{24}N_2ClFO_4.HCl$
Calculated: C,59.18;H,4.97;N,5.52;Cl,13.98
Found: C,59.12;H,4.91;N,5.56;Cl,14.50

P.R. = 67

Antag. of Actin-Myosin -100µM = 62%; 50µM = 23%

Antag. of MLCP -100µM =56%; 50µM = 18%

B.P. -dose 25 mg/kg =-22mm at 1.5 hours;-31mm at 4 hours.

EXAMPLE 24

4-(2,3-Dichloro-6-fluorophenyl)-1,4,5,6,7,8-hexahydro-2-methyl-5-oxo-1,7-naphthyridine-3-carboxylic acid 2-phenoxyethyl ester

(a) 2,3-Dichloro-6-fluorobenzaldehyde was prepared by a method analogous to the preparation of 2,3-difluorobenzaldehyde (Roe,A.M., et al., J.Med.Chem., 11,814, 1968).

Fifty-nine grams of 3,4-dichlorofluorobenzene were converted to 59.7g of 2,3-dichloro-6-fluorobenzaldehyde, m.p. 55-7°C.

| | |
|---|---|
| Analysis for: | $C_7H_3Cl_2FO$ |
| Calculated: | C,43.56;H,1.56; Cl,36.74 |
| Found: | C,43.17;H,1.69;Cl,36.96. |

(b) A mixture of 15.2g of 1-benzyl-3-hydroxy-5-oxo-2,3,4,6-tetrahydropyridine hydrate, 16.6g of 2-phenoxyethyl acetoacetate, 14.4g of 2,3-dichloro-6-fluorobenzaldehyde, 11.5g of ammonium acetate and 300 ml of methanol was refluxed for 6 hours. The solution was evaporated to dryness. The residue was dissolved in methylenechloride, extracted with water, then dried over magnesium sulfate. The solution was evaporated and the residue was crystallized from ethyl acetate. The mixture was filtered to obtain 16.4g of solid, m.p. 178-180°C. Treatment with hydrogen chloride in methanol afforded 14.3g of 4-(2,3-dichloro-6-fluorophenyl)-1,4,5,6,7,8-hexahydro-2-methyl-5-oxo-7-(phenylmethyl)-1,7-naphthyridine-3-carboxylic acid 2-phenoxyethyl ester hydrochloride, m.p. 185°C dec.

(c) The above hydrochloride (11.2g), 500 ml of methanol, 25ml of water, 5 ml of concentrated hydrochloric acid, and 0.5g of 10% palladium on carbon were shaken with hydrogen (45 psig initial pressure) for 4 hours. The catalyst was separated and the solution was evaporated to dryness in vacuo. The residue was recrystallized from methanol-diethyl to obtain 7.8g of the title compound as the hydrochloride,m.p. 235-6°C dec.

| | |
|---|---|
| Analysis for: | $C_{24}H_{21}N_2Cl_2FO_4$.HCl |
| Calculated: | C,54.61;H,4.20;N,5.31;Cl,20.15 |
| Found: | C,54.90;H,4.18;N,5.35;Cl,20.27 |

P.R. = 65

$IC_{25}$ = 1 × $10^{-5}$M

Antag. of Actin-Myosin -100µM=72±1%; 50µM=54±3%; 25µM=9±2%

Antag. of MLCP -100µM=68±1%; 50µM=50±2%; 25µM=8±2%.

B.P.-dose 25mg/kg=-50mm at 1.5 hours; -70mm at 4 hours
10mg/kg=-6mm at 1.5 hours; -26mm at 4 hours
5mg/kg=-17mm at 1.5 hours;-26mm at 4 hours.

## EXAMPLE 25

4-(2,3-dichloro-6-fluorophenyl)-1,4,5,6,7,8-hexahydro-2-methyl-5-oxo-1,7-naphthyridine-3-carboxylic acid 2-(2,3-dichlorophenoxy)ethyl ester

(a) Methanol (400 ml), 22.1g (0.1 mole) of 1-benzyl-3-hydroxy-5-oxo-1,2,3,6-tetrahydropyridine hydrate, 19.3g (0.1 mole) of 2,3-dichloro-6-fluorobenzaldehyde, 29.1g (0.1 mole) of 2-(2,3-dichlorophenoxy)ethyl acetoacetate and 16g (0.21 mole) of ammonium acetate were combined and heated to reflux. Solid began to precipitate after one-half hour, but refluxing was continued for 4 hours. The mixture was cooled to room temperature and filtered. The solid was washed with methanol, then dried in vacuo to obtain 34 grams - (52.3%) of 4-(2,3-Dichloro-6-fluorophenyl)-1,4,5,6,7,8-hexahydro-2-methyl-7-phenylmethyl-5-oxo-1,7-naphthyridine-3-carboxylic acid 2-(2,3-dichlorophenoxy)ethyl ester, m.p. 204-6°C.

Thirty-two grams of the above solid were suspended in methanol and saturated with hydrogen

chloride. The solution was filtered and evaporated to dryness in vacuo. Ethanol was added to the residue and re-evaporated. The solids were slurried with ethanol and filtered to obtain 35g, m.p. 231-5°C dec; HPLC = 100%.

A sample was recrystallized from methanol, m.p.232-5°C dec.

| | |
|---|---|
| Analysis for: | $C_{31}H_{25}N_2Cl_4FO_4$.HCl |
| Calculated: | C,54.21;H,3.82;N,4.08;Cl,25.81 |
| Found: | C,53.95;H,3.70;N,3.72;Cl,26.17. |

(b) Fifteen grams (0.218 mole) of the preceding 7-phenylmethyl protected compound as the hydrochloride, 1.5g of 10% Pd/C, 1200ml of methanol and 7 ml of concentrated hydrochloric acid were shaken with hydrogen at an initial pressure of 51 psig for 24 hours. The mixture was filtered and the filtrate was evaporated to dryness in vacuo. Ethanol was added to the residue and re-evaporated. The solid residue was slurried with ethanol and filtered to obtain 7.5g,m.p. 250-4°C with dec. The solid was stirred with 200 ml of refluxing ethanol, then filtered. The solid was dried in vacuo to obtain 6.2 grams (47.7%), m.p. 253-5°C with dec; HPLC = 97.4%.

| | |
|---|---|
| Analysis for: | $C_{24}H_{19}Cl_4FO_4$.HCl |
| Calculated: | C,48.30;H,3.38;N,4.70;Cl,29.71 |
| Found: | C,48.39;H,3.29;N,4.67;Cl,30.18. |

Three grams of the above solid were dissolved in hot methanol, treated with charcoal and filtered. The filtrate was evaporated in vacuo to the point of crystallization. After cooling in an ice bath, the solid was separated, washed with ethanol and dried in vacuo to obtain 1.8 grams, m.p. 253-5°C; HPLC = 98.5%.

Found: C,48.12;H,3.54;N,4.66;Cl,29.01

P.R. = 59

$IC_{25}$ = 0.7μM

Antag. of Actin-Myosin - 100μM=73±2.4%;50μM=67±5%; 25μM=55±2.5%;
10μM=34±7%

Antag. of MLCP - 100μM=67±2.3%;50μM=60±4%;25μM=48±3%; 10μM=32±8%

B.P.= dose 25mg/kg=-74mm at 1.5 hours, -70mm at 4 hours
5mg/kg=-18mm at 1.5 hours, -40mm at 4 hours.

## Claims

1. A compound of formula

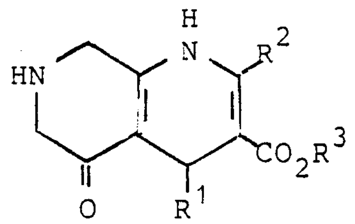

(I)

in which

$R^1$ is tetra-or penta-chloro, bromo or fluorophenyl or

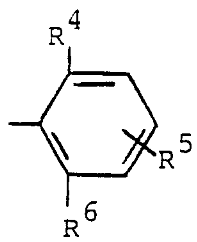

where $R^4$ and $R^6$ are, independently, hydrogen, alkyl of 1 to 6 carbon atoms, halo, trifluoromethyl, alkoxy of 1 to 6 carbon atoms, alkylthio of 1 to 6 carbon atoms, cyano or nitro; and $R^5$ is hydrogen, alkyl of 1 to 6 carbon atoms, halo, trifluoromethyl, cyano or nitro;

$R^2$ is alkyl of 1 to 6 carbon atoms; and $R^3$ is

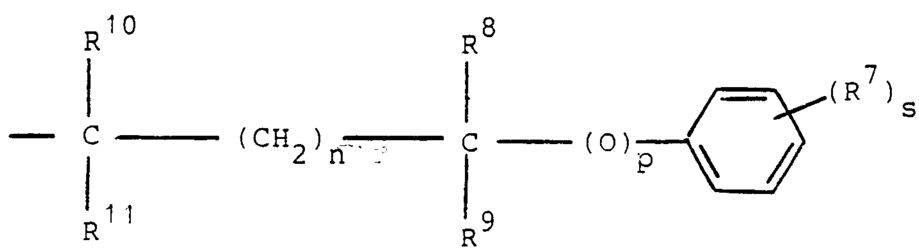

where $R^7$ is, independently, hydrogen, -Cl, -Br, -F, and no more than three of the groups $-CF_3$, alkyl of 1 to 6 carbon atoms or alkoxy of 1 to 6 carbon atoms;

$R^8$ is hydrogen, alkyl of 1 to 6 carbon atoms or alkoxy of 1 to 6 carbon atoms;

$R^9$, $R^{10}$ and $R^{11}$ are, independently, hydrogen or alkyl of 1 to 6 carbons atoms;

n is one of the integers 0, 1, 2, 3 or 4;

s is one of the integers 1, 2, 3, 4 or 5;

p is one of the integers 0 or 1, with the proviso that when $R^8$ is alkoxy, p is 0;

or $R^3$ is

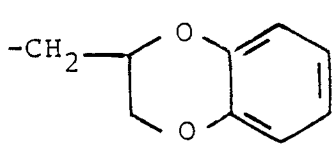

or

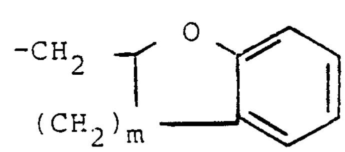

;

where m is one of the integers 1, 2, 3 or 4;

or a pharmaceutically acceptable salt thereof.

2. A compound as claimed in Claim 1 in which the group $R^1$ contains halogen substituents.

3. A compound as claimed in Claim 1 or Claim 2 in which $R^1$ is tetra-or penta-chloro, bromo or fluoro-phenyl or

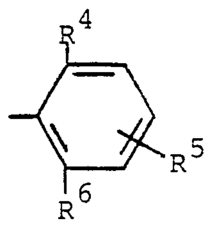

where $R^4$ and $R^6$ are halo and $R^5$ is hydrogen, alkyl of 1 to 6 carbon atoms, halo, trifluoromethyl, cyano or nitro.

4. A compound as claimed in Claim 1 in which $R^1$ is 3-chlorophenyl; 2,3-dichlorophenyl; 2,3,6-trifluorophenyl, 2-chloro-6-fluorophenyl, 2,3-dichloro-6-fluorophenyl, pentafluorophenyl, 3-chloro-2-methylphenyl, 2-chloro-6-fluoro-3-methylphenyl, 2-trifluoromethylphenyl or 2-methylphenyl.

5. A compound as claimed in any one of Claims 1 to 4 wherein $R^3$ is

(i) a group of formula

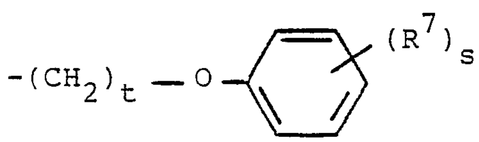

where t is an integer from 2 to 6 and $R^7$ and s are as defined in Claim 1;

or

(ii) a group of formula

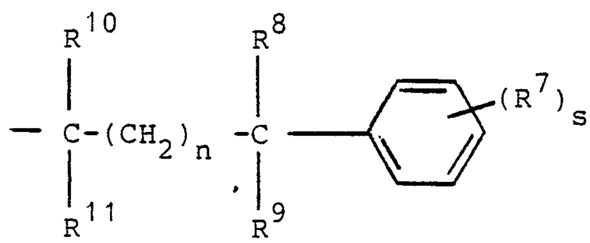

where n, s, $R^7$, $R^9$, $R^{10}$ and $R^{11}$ are as defined in Claim 1 and $R^8$ is alkoxy of 1 to 6 C atoms.

6. A compound as claimed in Claim 5 wherein t is 2 or 4 and/or $(R^7)_s$ is hydrogen or dichloro.

7. A compound as claimed in Claim 5 wherein $R^3$ is 2-methoxy-2-phenylethyl, 2-phenoxyethyl, 4-phenoxybutyl, 2-(2,3-dichlorophenoxy)ethyl, 1-methyl-2-phenoxyethyl, 2-methyl-2-phenoxypropyl or 2,3-dihydro-1,4-benzodioxin-2-ylmethyl.

8. A compound as claimed in Claim 1 which is one of the following: 4-(2,3-Dichlorophenyl)-1,4,5,6,7,8-hexahydro-2-methyl-5-oxo-1,7-naphthyridine-3-carboxylic acid 2-methoxy-2-phenylethyl ester;

1,4,5,6,7,8-Hexahydro-2-methyl-5-oxo-4-(pentafluorophenyl)-1,7-naphthyridine-3-carboxylic acid 2-phenoxyethyl ester;

(-)-1,4,5,6,7,8-Hexahydro-2-methyl-5-oxo-4-pentafluorophenyl)-1,7-naphthyridine-3-carboxylic acid 2-phenoxyethyl ester;

(+)-1,4,5,6,7,8-Hexahydro-2-methyl-5-oxo-4-(pentafluorophenyl)-1,7-naphthyridine-3-carboxylic acid 2-phenoxyethyl ester;

1,4,5,6,7,8-Hexahydro-2-methyl-5-oxo-4-(pentafluorophenyl)-1,7-naphthyridine-3-carboxylic acid 2-methoxy-2-phenylethyl ester;

1,4,5,6,7,8-Hexahydro-2-methyl-5-oxo-4-(pentafluorophenyl)-1,7-naphthyridine-3-carboxylic acid (R)-2-methoxy-2-phenylethyl ester;

1,4,5,6,7,8-Hexahydro-2-methyl-5-oxo-4-(pentafluorophenyl)-1,7-naphthyridine-3-carboxylic acid (S)-2-methoxy-2-phenylethyl ester;

1,4,5,6,7,8-Hexahydro-2-methyl-5-oxo-4R-(pentafluorophenyl)-1,7-naphthyridine-3-carboxylic acid (S)-2-methoxy-2-phenylethyl ester;

1,4,5,6,7,8-Hexahydro-2-methyl-5-oxo-4S-)pentafluorophenyl)-1,7-naphthyridine-3-carboxylic acid (S)-2-methoxy-2-phenylethyl ester;

4-(2-Chloro-6-fluorophenyl)-1,4,5,6,7,8-hexahydro-2-methyl-5-oxo-1,7-naphthyridine-3-carboxylic acid 2-methoxy-2-phenylethyl ester;

4-(3-Chloro-2-methylphenyl)-1,4,5,6,7,8-hexahydro-2-methyl-5-oxo-1,7-naphthyridine-3-carboxylic acid 2-methoxy-2-phenylethyl ester;

4-(3-Chlorophenyl)-1,4,5,6,7,8-hexahydro-2-methyl-5-oxo-1,7-naphthyridine-3-carboxylic acid 2-methoxy-2-phenylethyl ester;

1,4,5,6,7,8-Hexahydro-2-methyl-5-oxo-4-[2-(trifluoromethyl)phenyl]-1,7-naphthyridine-3-carboxylic acid 2-methoxy-2-phenylethyl ester;

1,4,5,6,7,8-Hexahydro-2-methyl-5-oxo-4-(pentafluorophenyl)-1,7-naphthyridine-3-carboxylic acid 1-methyl-2-phenoxyethyl ester;

1,4,5,6,7,8-Hexahydro-2-methyl-5-oxo-4-(pentafluorophenyl)-1,7-naphthyridine-3-carboxylic acid 2,3-dihydro-1,4-benzodioxin-2-ylmethyl ester;

1,4,5,6,7,8-Hexahydro-2-methyl-4-(2-methylphenyl)-5-oxo-1,7-naphthyridine-3-carboxylic acid 2-phenoxyethyl ester;

1,4,5,6,7,8-Hexahydro-2-methyl-5-oxo-4-(2,3,6-trifluorophenyl)-1,7-naphthyridine-3-carboxylic acid 2-phenoxyethyl ester;

4-(3-Chloro-2-methylphenyl)-1,4,5,6,7,8-hexahydro-2-methyl-5-oxo-1,7-naphthyridine-3-carboxylic acid 2-phenoxyethyl ester;

1,4,5,6,7,8-Hexahydro-2-methyl-5-oxo-4-(pentafluorophenyl)-1,7-naphthyridine-3-carboxylic acid 2-phenylethyl ester;

4-(2,3-Dichlorophenyl)-1,4,5,6,7,8-hexahydro-2-methyl-5-oxo-1,7-naphthyridine-3-carboxylic acid 2-phenoxyethyl ester;

1,4,5,6,7,8-Hexahydro-2-methyl-5-oxo-4-(pentafluorophenyl)-1,7-naphthyridine-3-carboxylic acid 2-methyl-2-phenoxypropyl ester;

1,4,5,6,7,8-Hexahydro-2-methyl-5-oxo-4-(pentafluorophenyl)-1,7-naphthyridine-3-carboxylic acid 4-phenoxybutyl ester;

1,4,5,6,7,8-Hexahydro-2-methyl-5-oxo-4-(pentafluorophenyl)-1,7-naphthyridine-3-carboxylic acid 2-(2,3-dichlorophenoxy)ethyl ester;

4-(2-Chloro-6-fluoro-3-methylphenyl)-1,4,5,6,7,8-hexahydro-2-methyl-5-oxo-1,7-naphthyridine-3-carboxylic acid 2-phenoxyethyl ester;

4-(2,3-Dichloro-6-fluorophenyl)-1,4,5,6,7,8-hexahydro-2-methyl-5-oxo-1,7-naphthyridine-3-carboxylic acid 2-phenoxyethyl ester; or a pharmaceutically acceptable salt therof.

9. 4-(2,3-Dichloro-6-fluorophenyl)-1,4,5,6,7,8-hexahydro-2-methyl-5-oxo-1,7-naphthyridine-3-carboxylic acid 2-(2-3-dichlorophenoxy)ethyl ester or a pharmaceutically acceptable salt thereof.

10. A compound of formula I as claimed in any one of the preceding claims which is in the form of a salt from an acid selected from hydrochloric, hydrobromic, phosphoric, sulfuric, sulfonic, nitric, methylsulfonic, acetic, maleic, succinic, fumaric, tartaric, citric, salicylic, lactic and naphthalenesulfonic acid.

11. A process for preparing a compound as claimed in Claim 1 which comprises

(a) deprotecting a compound of formula II

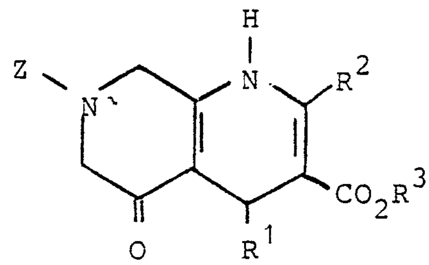

(II)

wherein $R^1$,$R^2$ and $R^3$ are as defined in Claim 1 and Z is a methyl group substituted by 1 to 3 aryl groups;

or b) transesterifying a compound of formula III

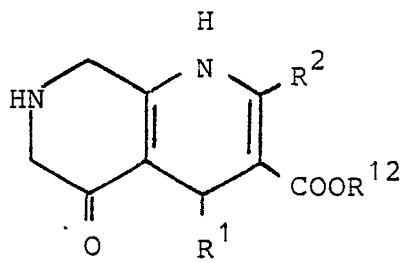

III

where $COOR^{12}$ is an ester group other than that desired in the end product to replace the $OR^{12}$ group by $OR^3$ and give a corresponding compound of formula I;

or c) converting a compound of formula I to a pharmaceutically acceptable salt thereof by addition of an acid to the free base form or by metathetical displacement with an acid;

or d) converting an acid addition salt of a compound of formula I to the free base form by addition of base,

or e) isolating from a mixture of isomers of a compound of formula I as claimed in Claim 1, an enantiomeric or diastereomeric form thereof.

12. A process for the preparation of a compound as claimed in Claim 1 which comprises (i) a Michael addition -cyclic condensation of an oxoenamine or precursor thereof to a Knoevenagel condensation product of an appropriately substituted aldehyde and an alkanoylacetic ester or a 1-arylmethyl-3-hydroxy-5-oxo-2,3,4,6-tetrahydropyridine, or precursors of said Knoevenagel condensation product, and (ii) removal of the N-arylmethyl protecting group from the product of step (i).

13. A compound of formula I as claimed in any one of Claims 1 to 10 for use as a pharmaceutical.

14. A pharmaceutical composition comprising a compound of formula I as claimed in any one of Claims 1 to 10 and a pharmaceutically acceptable carrier.

15. A pharmaceutical carrier according to Claim 14 in the form of a tablet or capsule.

16. A compound of formula II

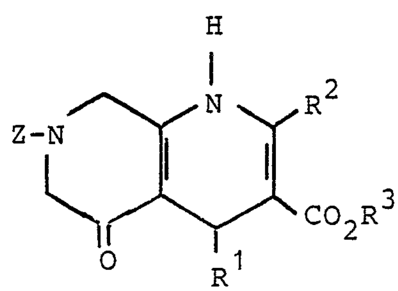

II

or acid addition salt thereof,

wherein $R^1$, $R^2$ and $R^3$ are as defined in any one of Claims 1 to 7 and Z is a methyl group substituted by 1 to 3 aryl groups.

17. A compound of formula II as claimed in Claim 16 which is the N-benzyl precursor for a compound as claimed in Claim 8 or Claim 9.

18. A process for preparing a compound of formula II as claimed in Claim 16 which comprises a Michael addition -cyclic condensation according to Claim 12 step (i).

Claims for the Contracting State:AT

1. A process for preparing a compound of formula

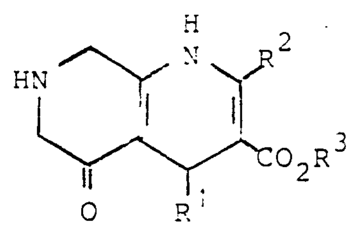

(I)

in which

$R^1$ is tetra-or penta-chloro, bromo or fluorophenyl or

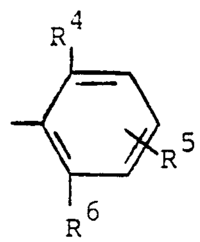

where $R^4$ and $R^6$ are, independently, hydrogen, alkyl of 1 to 6 carbon atoms, halo, trifluoromethyl, alkoxy of 1 to 6 carbon atoms, alkylthio of 1 to 6 carbon atoms, cyano or nitro; and $R^5$ is hydrogen, alkyl of 1 to 6 carbon atoms, halo, trifluoromethyl, cyano or nitro;

$R^2$ is alkyl of 1 to 6 carbon atoms; and $R^3$ is

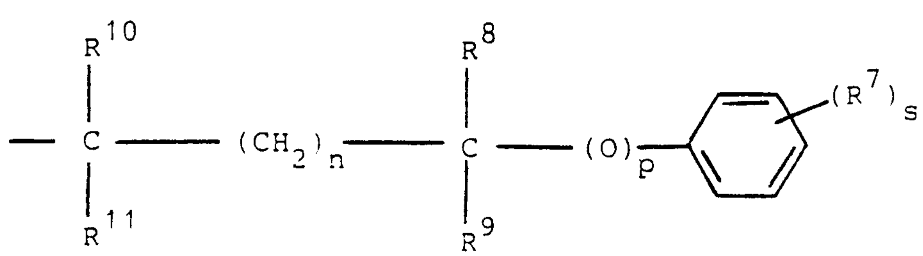

where $R^7$ is, independently, hydrogen, -Cl, -Br, -F, and no more than three of the groups $-CF_3$, alkyl of 1 to 6 carbon atoms or alkoxy of 1 to 6 carbon atoms;

$R^8$ is hydrogen, alkyl of 1 to 6 carbon atoms or alkoxy of 1 to 6 carbon atoms;

$R^9$, $R^{10}$ and $R^{11}$ are, independently, hydrogen or alkyl of 1 to 6 carbon atoms;

n is one of the integers 0,1,2,3 or 4;

s is one of the integers 1,2,3,4 or 5;

p is one of the integers 0 or 1, with the proviso that when $R^8$ is alkoxy, p is 0;

or $R^3$ is

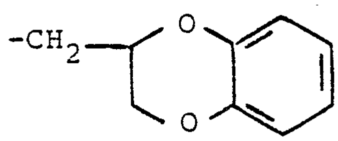

or

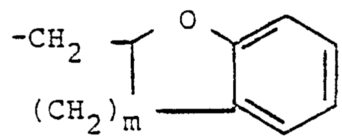

;

where m is one of the integers 1,2,3 or 4;

or a pharmaceutically acceptable salt thereof, which comprises

a) deprotecting a compound of formula II

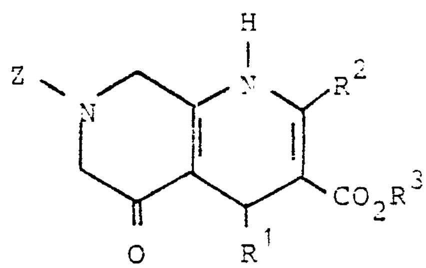

(II)

wherein $R^1$, $R^2$ and $R^3$ are as defined above and Z is a methyl group substituted by 1 to 3 aryl groups;

or b) transesterifying a compound of formula III

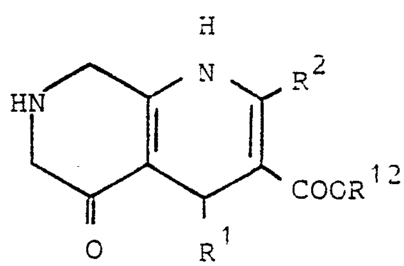

III

where $COOR^{12}$ is an ester group other than that desired in the end produce to replace the $OR^{12}$ group by $OR^3$ and give a corresponding compound of formula I;

or c) converting a compound of formula I to a pharmaceutically acceptable salt thereof by addition of an acid to the free base form or by metathetical displacement with an acid;

or d) converting an acid addition salt of a compound of formula I to the free base form by addition of base,

or e) isolating from a mixture of isomers of a compound of formula I as claimed in Claim 1, an enantiomeric or diastereomeric form thereof.

2. A process as claimed in Claim 1 in which the compound of formula II is prepared by a process which comprises a Michael addition -cyclic condensation of an oxoamine or precursor thereof to a knoevenagel condensation product of an appropriately substituted aldehyde and an alkanoylacetic ester or a 1-arylmethyl-3-hydroxy-5-oxo-2,3,4,6-tetrahydropyridine, or precursors of said Knoevenagel condensation product.

3. A process (a) according to Claim 1 in which the compound of formula II wherein Z is benzyl is prepared by one of the following

a) reacting together a compound of formula

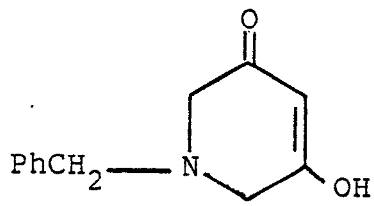

with (i) an ammonia source and compounds of formulae

$R^1CHO$ and $R^2COCH_2CO_2R^3$

or (ii) compounds of formulae

$R^1CHO$ and $H_2NCR^2=CHCO_2R^3$

or (iii) an ammonia source and a compound of formula

$R^1CH = C(CO_2R^3)COR^2$

or (b) reacting together a compound of formula

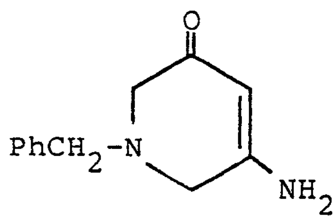

with (i) compounds of formulae

$R^1CHO$ and $R^2COCH_2CO_2R^3$

or (ii) a compound of formula

$R^1CH=C(CO_2R^3)COR^2$

or (c) reacting a compound of formula

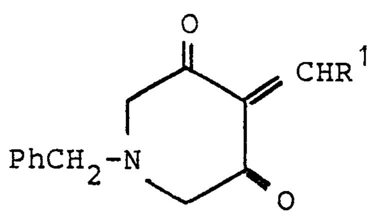

with a compound of formula

$H_2NCR^2=CHCO_2R^3$ ;

in which formulae $R^1$, $R^2$ and $R^3$ are as defined in Claim 1;

and the deprotection is carried out by catalytic hydrogenation to give a compound of formula I as defined in Claim 1.

4. A process as claimed in any one of Claims 1 to 3 in which the group $R^1$ in the compound of formula I prepared contains halogen substituents.

5. A process as claimed in any one of Claims 1 to 3 in which $R^1$ in the compound of formula I prepared is tetra-or penta-chloro, bromo or fluorophenyl or

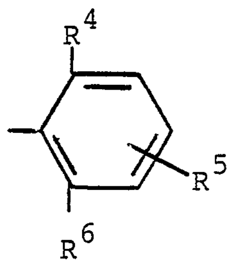

where $R^4$ and $R^6$ are halo and $R^5$ is hydrogen, alkyl of 1 to 6 carbon atoms, halo, trifluoromethyl, cyano or nitro.

6. A process as claimed in any one of Claims 1 to 3 in which $R^1$ is 3-chlorophenyl; 2,3-dichlorophenyl; 2,3,6-trifluorophenyl, 2-chloro-6-fluorophenyl, 2,3-dichloro-6-fluorophenyl, pentafluorophenyl, 3-chloro-2-methylphenyl, 2-chloro-6-fluoro-3-methylphenyl, 2-trifluoromethylphenyl or 2-methylphenyl.

7. A process as claimed in any one of Claims 1 to 6 wherein $R^3$ is

(i) a group of formula

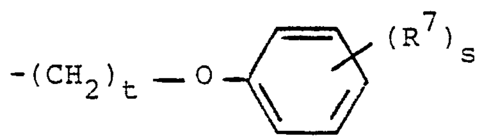

where t is an integer from 2 to 6 and $R^7$ and s are as defined in Claim 1;

or

(ii) a group of formula

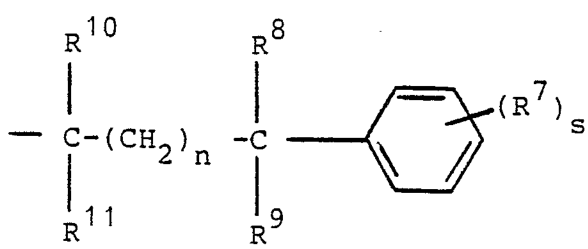

where n, s, $R^7$, $R^9$, $R^{10}$ and $R^{11}$ are as defined in Claim 1 and $R^8$ is alkoxy of 1 to 6 C atoms.

8. A process as claimed in Claim 7 wherein t is 2 or 4 and/or $(R^7)_s$ is hydrogen or dichloro.

9. A process as claimed in Claim 1 in which $R^3$ in the compound of formula I prepared is 2-methoxy-2-phenylethyl, 2-phenoxyethyl, 4-phenoxybutyl, 2-(2,3-dichlorophenoxy)ethyl, 1-methyl--2-phenoxyethyl, 2-methyl-2-phenoxypropyl or 2,3-dihydro-1,4-benzodioxin-2-ylmethyl.

10. A process as claimed in Claim 1 in which the compound prepared is selected from one of the following

4-(2,3-dichlorophenyl)-1,4,5,6,7,8-hexahydro-2-methyl-5-oxo-1,7-naphthyridine-3-carboxylic acid 2-methoxy-2-phenylethyl ester.

1,4,5,6,7,8-hexahydro-2-methyl-5-oxo-4-(pentafluorophenyl)-1,7-naphthyridine-3-carboxylic acid 2-phenoxyethyl ester;

(-)-1,4,5,6,7,8-hexahydro-2-methyl-5-oxo-4-pentafluorophenyl)-1,7-naphthyridine-3-carboxylic acid 2-phenoxyethyl ester;

(+)-1,4,5,6,7,8-hexahydro-2-methyl-5-oxo-4-(pentafluorophenyl)-1,7-naphthyridine-3-carboxylic acid 2-phenoxyethyl ester;

1,4,5,6,7,8-hexahydro-2-methyl-5-oxo-4-(pentafluorophenyl)-1,7-naphthyridine-3-carboxylic

acid 2-methoxy-2-phenylethyl ester;

1,4,5,6,7,8-hexahydro-2-methyl-5-oxo-4-(pentafluorophenyl)-1,7-naphthyridine-3-carboxylic acid (R)-2-methoxy-2-phenylethyl ester;

1,4,5,6,7,8-hexahydro-2-methyl-5-oxo-4-(pentafluorophenyl)-1,7-naphthyridine-3-carboxylic acid (S)-2-methoxy-2-phenylethyl ester;

1,4,5,6,7,8-hexahydro-2-methyl-5-oxo-4R-(pentafluorophenyl)-1,7-naphthyridine-3-carboxylic acid (S)-2-methoxy-2-phenylethyl ester

1,4,5,6,7,8-hexahydro-2-methyl-5-oxo-4S-(pentafluorophenyl)-1,7-naphthyridine-3-carboxylic acid (S)-2-methoxy-2-phenylethyl ester

4-(2-chloro-6-fluorophenyl)-1,4,5,6,7,8-hexahydro-2-methyl-5-oxo-1,7-naphthyridine-3-carboxylic acid 2-methoxy-2-phenylethyl ester

4-(3-chloro-2-methylphenyl)-1,4,5,6,7,8-hexahydro-2-methyl-5-oxo-1,7-naphthyridine-3-carboxylic acid 2-methoxy-2-phenylethyl ester

4-(3-chlorophenyl)-1,4,5,6,7,8-hexahydro-2-methyl-5-oxo-1,7-naphthyridine-3-carboxylic acid 2-methoxy-2-phenylethyl ester

1,4,5,6,7,8-hexahydro-2-methyl-5-oxo-4-[2-(trifluoromethyl)phenyl]-1,7-naphthyridine-3-carboxylic acid 2-methoxy-2-phenylethyl ester

1,4,5,6,7,8-hexahydro-2-methyl-5-oxo-4-(pentafluorophenyl)-1,7-naphthyridine-3-carboxylic acid 1-methyl-2-phenoxyethyl ester;

1,4,5,6,7,8-hexahydro-2-methyl-5-oxo-4-(pentafluorophenyl)-1,7-naphthyridine-3-carboxylic acid 2,3-dihydro-1,4-benzodioxin-2-ylmethyl ester;

1,4,5,6,7,8-hexahydro-2-methyl-4-(2-methylphenyl)-5-oxo-1,7-naphthyridine-3-carboxylic acid 2-phenoxyethyl ester;

1,4,5,6,7,8-hexahydro-2-methyl-5-oxo-4-(2,3,6-trifluorophenyl)-1,7-naphthyridine-3-carboxylic acid 2-phenoxyethyl ester

4-(3-chloro-2-methylphenyl)-1,4,5,6,7,8-hexahydro-2-methyl-5-oxo-1,7-naphthyridine-3-carboxylic acid 2-phenoxyethyl ester

1,4,5,6,7,8 hexahydro-2-methyl-5-oxo-4-(pentafluorophenyl)-1,7-naphthyridine-3-carboxylic acid 2-phenylethyl ester

4-(2,3-dichlorophenyl)-1,4,5,6,7,8-hexahydro-2-methyl-5-oxo-1,7-naphthyridine-3-carboxylic acid 2-phenoxyethyl ester

1,4,5,6,7,8-Hexahydro-2-methyl-5-oxo-4-(pentafluorophenyl)-1,7-naphthyridine-3-carboxylic acid 2-methyl-2-phenoxypropyl ester;

1,4,5,6,7,8-Hexahydro-2-methyl-5-oxo-4-(pentafluorophenyl)-1,7-naphthyridine-3-carboxylic acid 4-phenoxybutyl ester;

1,4,5,6,7,8-Hexahydro-2-methyl-5-oxo-4-(pentafluorophenyl)-1,7-naphthyridine-3-carboxylic acid 2-(2,3-dichlorophenoxy)ethyl ester;

4-(2-Chloro-6-fluoro-3-methylphenyl)-1,4,5,6,7,8-hexahydro-2-methyl-5-oxo-1,7-naphthyridine-3-carboxylic acid 2-phenoxyethyl ester or

4-(2,3-Dichloro-6-fluorophenyl)-1,4,5,6,7,8-hexahydro-2-methyl-5-oxo-1,7-naphthyridine-3-carboxylic acid 2-phenoxyethyl ester; or a pharmaceutically acceptable salt thereof.

11. A process as claimed in Claim 1 in which the compound prepared is 4-(2,3-dichloro-6-fluorophenyl)-1,4,5,6,7,8-hexahydro-2-methyl-5-oxo-1,7-naphthyridine-3-carboxylic acid 2-(2-3-dichlorophenoxy)ethyl ester or a pharmaceutically acceptable salt thereof.

12. A process as claimed in any one of the preceding claims in which the compound of formula I prepared is in the form of a salt from an acid selected from hydrochloric, hydrobromic, phosphoric, sulfuric, sulfonic, nitric, methylsulfonic, acetic, maleic, succinic, fumaric, tartaric, citric, salicyclic, lactic and naphthalenesulfonic acid.

13. A process for preparing a pharmaceutical composition which comprises bringing a compound of formula I as defined in Claim 1 or a pharmaceutically acceptable salt thereof into association with a pharmaceutically acceptable carrier.

14. A process as claimed in Claim 13 in which the composition is produced in the form of a tablet or capsule.

15. A process for preparing a compound of formula II

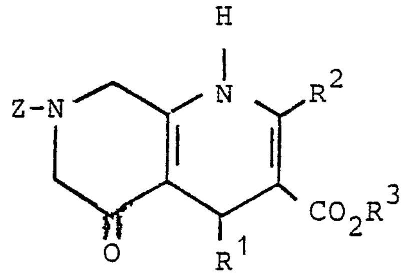

II

or acid addition salt thereof,

wherein $R^1$, $R^2$ and $R^3$ are as defined in Claim 1 and Z is a methyl group substituted by 1 to 3 aryl groups, which comprises a Michael addition -cyclic condensation of an oxoenamine or precursor thereof to a Knoevenagel condensation product of an appropriately substituted aldehyde and an alkanoylacetic ester or a 1-arylmethyl-3-hydroxy-5-oxo-2,3,4,6-tetrahydropyridine, or precursors of said Knoevenagel condensation product.

16. A process for preparing a compound of formula II as defined in Claim 15 where Z is benzyl which comprises one of the following

a) reacting together a compound of formula

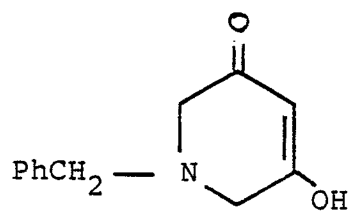

with (i) an ammonia source and compounds of formulae

$R^1CHO$ and $R^2COCH_2CO_2R^3$

or (ii) compounds of formulae

$R^1CHO$ and $H_2NCR^2=CHCO_2R^3$

or (iii) an ammonia source and a compound of formula

$R^1CH = C(CO_2R^3)COR^2$

or (b) reacting together a compound of formula

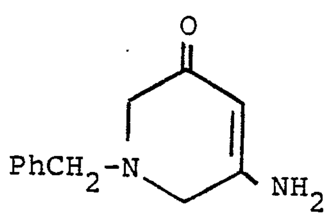

with (i) compounds of formulae

$R^1CHO$ and $R^2COCH_2CO_2R^3$

or (ii) a compound of formula

$R^1CH=C(CO_2R^3)COR^2$

or (c) reacting a compound of formula

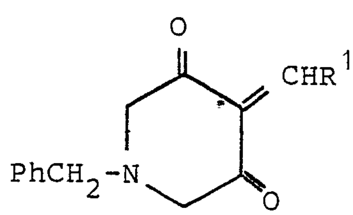

with a compound of formula

$H_2NCR^2=CHCO_2R^3$ ;

in which formulae $R^1$, $R^2$,$R^3$ are as defined in any one of Claims 1 and 4 to 7.

European Patent Office

# EUROPEAN SEARCH REPORT

Application number

EP 86305734.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US - A - 4 321 384 (SULKOWSKI)<br>* Claims 1,18; column 1, lines 59-66; column 2, line 57 - column 3, line 10 *<br>-- | 1,11-18 | C 07 D 471/04<br>A 61 K 31/435<br>//(C 07 D 471/04<br>C 07 D 221:00<br>C 07 D 221:00) |
| D,A | US - A - 4 365 063 (SULKOWSKI)<br>* Column 1, line 60 - column 3, line 17 *<br>---- | 1,11-18 | |
| | | | **TECHNICAL FIELDS SEARCHED (Int. Cl.4)** |
| | | | C 07 D 471/00 |

The present search report has been drawn up for all claims

| Place of search | Date of completion of the search | Examiner |
|---|---|---|
| VIENNA | 06-10-1986 | ONDER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document